# FASCICULE DE BREVET EUROPEEN

(11) **EP 0 698 099 B1**
(45) Date de publication et mention de la délivrance du brevet: **13.07.2005**
(21) Numéro de dépôt: 94916287.9
(22) Date de dépôt: 13.05.1994
(51) Int. Cl.: C12N 15/30, C12N 1/10, A61K 39/008, C12P 21/08, G01N 33/569, A61K 49/00, G01N 33/60

(54) **PROCEDE DE CULTURE IN VITRO DE DIFFERENTS STADES DE PARASITES TISSULAIRES**
IN VITRO KULTURPROZESS FÜR VERSCHIEDENE STADIEN VON GEWEBEPARASITEN
METHOD FOR THE CULTURE IN VITRO OF DIFFERENT STAGES OF TISSUE PARASITES

(30) Priorité: 13.05.1993 FR 9305779
(43) Date de publication de la demande: 28.02.1996
(73) Titulaire: Institut de Recherche pour le Développement ( IRD), 75480 Paris Cedex 10 (FR)
(72) Inventeur: LEMESRE, Jean-Loup, F-34380 Saint-Martin-De-Londres (FR)
(74) Mandataire: Peaucelle, Chantal
(86) Numéro de dépôt international: PCT/FR1994/000577
(87) Numéro de publication internationale: WO 1994/026899

(56) Documents cités:
- EP-A- 0 420 635
- WO-A-83/01785
- WO-A-86/02098
- WO-A-93/03167
- FR-A- 1 518 449
- US-A- 3 911 097
- US-A- 3 993 743
- US-A- 4 687 666
- ACTA TROPICA vol. 45, no. 2 , 1988 pages 99 - 108 LEMESRE, J.L. ET AL. 'Requirements of defined cultivation conditions for standard growth of Leishmania promastigotes in vitro'
- MOLECULAR AND BIOCHEMICAL PARASITOLOGY vol. 58, no. 2 , 17 Mars 1993 pages 345 - 354 JOSHI, M. ET AL. 'Cloning and characterization of differentially expressed genes from in-vitro-grown "amastigotes" of leishmania donovani'
- PARASITOLOGY vol. 83, no. 3 , Décembre 1981 pages 529 - 541 HART, D.T. ET AL. 'Transformation in vitro of Leishmania mexicana amastigotes to promastigotes: nutritional requirements and the effects of drugs'
- PARASITE IMMUNOLOGY vol. 11, no. 3 , Mai 1989 pages 197 - 209 KWEIDER, M. ET AL. 'Development of metacyclic Leishmania promastigotes is associated with the increasing expression of GP65, the major surface antigen'
- ACTA TROPICA vol. 45, no. 2 , 1988 pages 109 - 126 O'DALY, J.A. & RODRIGUEZ, M.B. 'Differentiation growth requirements of several Leishmania spp. in chemically defined culture media'
- EXPERIMENTAL PARASITOLOGY vol. 64, no. 3 , Décembre 1987 pages 368 - 375 DE CASTRO, S. ET AL. 'Trypanosoma cruzi : adrenergic modulation of cyclic AMP role in proliferation and differantiation of amastigotes in vitro'
- CHEMOSPHERE vol. 19, no. 10/1 , 1989 pages 1743 - 1748 VELASCO,J.R. ET AL. 'A survey of culture media of Trypanosoma cruzi amastigote forms from infected Vero cells'
- CHEMOSPHERE vol. 21, no. 1-2 , 1990 , UK pages 263 - 268 VELASCO, J.R. ET AL. 'In vitro survival of amastigote forms of Trypanosoma cruzi in media conditioned by Vero cells'
- ANNALES DES SOCIETES BELGES DE MEDECINE TROPICALE vol. 49, no. 4 , 1969 pages 331 - 340 JADIN, J. & LE RAY, D. 'Acquisitions récentes dans les techniques de culture des Trypanosomes africains'
- O'DALY ET AL EXPERIMENTAL PARASITOLOGY vol. 64, no. 1, 1987, pages 78 - 87

## Description

L'invention a pour objet un procédé de culture in vitro, de différents stades du cycle évolutif d'un parasite. Elle vise également les formes parasitaires obtenues et leurs applications biologiques.

Par culture, on entend dans la description qui suit et les revendications, aussi bien l'adaptation de la forme parasitaire par passages successifs dans un milieu donné, que la différenciation complète quand elle a lieu pour l'adaptation et la culture proprement dite de la forme parasitaire.

On sait que les parasites constituent un véritable fléau provoquant, par l'intermédiaire de vecteurs, l'infection de millions d'hommes et d'animaux.

Ainsi, les leishmanioses, répandues dans le monde entier, sont provoquées par des protozoaires du genre Leishmania qui sont transmis le plus généralement par un moustique, le phlébotome. On distingue classiquement, selon leur localisation géographique, les leishmanioses de l'Ancien Monde et celles du Nouveau Monde. Elles présentent des formes cliniques très diverses qui diffèrent sensiblement par leur gravité et leur retentissement sur la santé. On distingue les leishmanioses cutanées, cutanéo-muqueuses (attaquant les muqueuses nasales, buccales et celles des oreilles) et les leishmanioses viscérales.

Comme autre parasite ayant un effet dévastateur, on citera également le Trypanosoma cruzi, responsable de la maladie de Chagas. En Amérique du Sud, il provoque l'infection de millions de personnes. On dénombre jusqu'à plus de 150 espèces d'animaux sauvages ou domestiques qui peuvent servir d'hôtes au parasite qui est transmis à l'homme par une punaise, à savoir un triatome, qui se nourrit de sang.

L'infection peut passer inaperçue pendant des années jusqu'à ce que les trypanosomes s'attaquent au système nerveux, au coeur ou à l'appareil digestif.

Le cycle évolutif de bon nombre de parasites comporte différents stades parasitaires. Celui des leishmanies, par exemple, comprend deux stades présentant d'importantes différences à la fois au plan structural, morphologique, biochimique, immunologique et physiologique, à savoir
- chez l'insecte vecteur, une forme flagellée, appelée promastigote, qui s'y multiplie par scissiparité avant d'acquérir sa forme infectante, pour l'hôte mammifère, encore appelée métacyclique,
- chez l'hôte mammifère, un stade non flagellé, appelé amastigote, qui parasite exclusivement les cellules phagocytaires mononucléées.

La différenciation en amastigotes survient après l'attachement et la pénétration des promastigotes dans les monocytes. Mais seules les formes amastigotes persistent et se multiplient à l'intérieur du phagolysosome des macrophages de l'hôte infecté.

Chez T. cruzi, ce cycle comporte trois stades parasitaires différents, une forme épimastigote, forme de multiplication de l'insecte vecteur, et les formes amastigote et trypomastigote, qui sont présentes chez l'hôte infecté.

Actuellement, la plupart des recherches menées sur le diagnostic de ces parasitoses et l'élaboration de vaccins sont réalisées sur la forme promastigote dont la production en culture est facile et rapide.

Or la seule forme présente chez l'hôte infecté est la forme amastigote qui, en persistant tout au long de l'infection, suscite la réponse immune et participe au développement de la pathologie. On mesurera le danger d'extrapoler systématiquement des résultats expérimentaux obtenus avec les formes promastigotes de culture dans l'objectif d'études immunoprophylactiques, diagnostiques ou thérapeutiques ciblées sur les formes amastigotes.

Pour répondre à ce problème, divers auteurs se sont intéressés à l'obtention de formes amastigotes. On a ainsi rapporté l'obtention d'amastigotes à partir de tissus d'animaux expérimentalement infectés ou à partir de cultures de macrophages infectés. Mais il s'agit de méthodes d'isolement longues et fastidieuses, qui en outre aboutissent à l'obtention d'un nombre limité de parasites, souvent dégradés, et qui sont incapables de se multiplier et de survivre plus de 2 à 3 jours.

De plus, de telles formes amastigotes sont contaminées par des cellules, fragments et molécules dérivés des macrophages, des tissus ou du plasma de l'hôte, désignés ci-après par "contaminants cellulaires", limitant ou rendant impossible la réalisation de bon nombre d'études.

Des procédés de culture en conditions axéniques, c'est-à-dire en l'absence de tout contaminant cellulaire, ont été proposés pour quelques espèces de leishmanies et pour T.cruzi, mais ne permettent pas de disposer d'une source abondante, en continu, de formes amastigotes, et n'apparaissent pas généralisables à un grand nombre d'espèces et à des germes différents.

L'invention a pour but de résoudre les inconvénients ci-dessus et de fournir des modèles expérimentaux plus satisfaisants en produisant les stades parasitaires souhaités dans des milieux de culture spécifiques, de composition simplifiée, totalement définie.

L'invention vise spécialement à fournir un procédé généralisable à un grand nombre d'espèces d'un parasite donné, permettant de produire de manière continue et en quantités illimitées des populations homogènes d'un stade parasitaire donné.

Elle vise plus particulièrement un procédé permettant d'obtenir ces stades sous une forme débarassée de tout contaminant cellulaire et sérique, ayant la particularité de ne contenir aucune macromolécule. Par macromolécules, on entend dans la description et les revendications qui suivent des molécules non dialysables à un seuil de coupure de 3 kDa, c'est-à-dire ayant un poids moléculaire apparent supérieur à 3 kDa (par exemple protéine sérique comme l'albumine).

Elle vise également la réalisation in vitro du cycle évolutif complet des parasites en conditions axénique et asérique.

Selon un autre aspect, l'invention a pour but les nouvelles formes parasitaires obtenues, correspondant aux différents stades du cycle des parasites et, pour chaque stade, aux différentes phases de leur croissance.

Selon encore un autre aspect, l'invention vise les applications des stades parasitaires obtenus et des produits générés ou isolés à partir de ces stades, notamment dans le domaine du diagnostic de parasitoses, de l'immunoprophylaxie et du criblage d'activités médicamenteuses.

Le procédé selon l'invention, pour la culture in vitro de différents stades de parasites tissulaires, tels que les leishmanies, T. cruzi, ou encore les hématoprotozoaires est caractérisé en ce qu'il est réalisé dans un milieu de culture monophasique liquide axénique et asérique, dépourvu de macromolécules (non dialysables à un seuil de coupure de 3kDa) et que pour l'obtention de formes amastigotes, ce milieu est tamponné à un pH de 5,5 à 6,5 et présente une osmolarité d'au moins 400 milli osmoles/kg de liquide, et en particulier de 400 à 550 milli osmoles/kg de liquide, ou que, pour l'obtention de formes promastigotes, ce milieu est tamponné à un pH de 7 à 7,5 et présente une osmolarité d'au moins 300 milli osmoles/kg de liquide, et en particulier de 300 à 380 milli osmoles/kg de liquide.

La valeur du pH de ces milieux, dans l'intervalle indiqué ci-dessus, assure un contrôle rigoureux des conditions de culture.

Dans le cas de la culture d'amastigotes, lorsque le pH est supérieur à 6,5, on note en effet une tendance à la retransformation des amastigotes en promastigotes et lorsqu'il est inférieur à 5,5, on observe une mauvaise croissance.

Selon un mode préféré de l'invention, pour l'obtention de formes amastigotes, on utilise un milieu de culture comprenant un milieu de base, élaboré essentiellement à partir :
- d'au moins un milieu de culture de cellules d'insectes additionné de sels inorganiques, du type des sels de Hanks,
- de produits sources, d'acides aminés, comme la L-glutamine et des extraits de graine de soja,
- des sucres, comme le D-glucose.

Comme extrait de graines de soja, on utilise avantageusement celui commercialisé sous la marque trypto caséine soja ^{R}.

Le milieu de culture de cellules d'insectes est avantageusement le milieu 199 H commercialisé par GIBCO.

Différentes compositions de ce milieu 199H ^{R} sont données dans le catalogue GIBCO BRL page 48, édition 1992.

Une composition spécialement préférée pour l'élaboration des milieux de base porte la référence 042-01181 à la page 48 de ce catalogue, édition 1992. On utilise plus spécialement le milieu 199H M, auquel on ajoute NaHCO₃ et de la L-glutamine.

Ce milieu auquel les composés ci-dessus sont ajoutés est avantageusement tamponné, par exemple avec un tampon comme l'HEPES.

Une composition préférée de milieu de base comprend plusieurs milieux de culture de cellules d'insectes.

Un milieu de base de ce type résulte de l'addition au milieu 199 H tel que commercialisé par Gibco, auquel ont été ajoutés les composés mentionnés ci-dessus, d'un autre milieu tel que le milieu 199 H modifié tel que commercialisé par Flow. Une composition de ce milieu est donné dans le catalogue Flow Laboratories, 1992 sous la référence 14230-54. Avant de l'ajouter à la préparation initiale, ce dernier milieu 199 H modifié est soumis à un traitement thermique.

Les milieux de base définis ci-dessus sont nouveaux et, en tant que tels, font également partie de l'invention. Ils peuvent être conservés pendant plusieurs mois à -20°C.

Pour éviter l'oxydation des stades parasitaires, ce milieu de base est additionné au moment de l'utilisation d'agents anti-oxydants tels que l'hémine, qui présente également l'avantage de constituer une source de fer et d'agents à effet réducteur comme le glutathion réduit. On ajoute également avantageusement des vitamines. Un mélange de vitamines approprié comprend la biotine, le panthoténate de calcium D, le chlorure de choline, l'acide folique, le i-inositol, le nicotinamide, l'acide para-aminobenzoïque, le chlorhydrate de pyridoxine, la riboflavine, le chlorhydrate de thiamine, la vitamine B12, utilisés en partie et avantageusement en totalité.

Les milieux de culture résultants sont avantageusement caractérisés en ce qu'ils sont dépourvus, en tant qu'additifs, de nucléotides. Ils peuvent être conservés à +4°C pendant environ deux semaines sans altération de leurs propriétés.

L'utilisation des milieux de culture résultants, comme illustré par les exemples donnés ci-après, permettent de produire des formes amastigotes, capables de se multiplier massivement in vitro, de manière continue.

Ces milieux axéniques et asériques présentent l'avantage d'être dépourvus de toutes macromolécules et notamment de celles présentes dans le sérum de veau foetal et/ou provenant des cellules hôtes, qui peuvent masquer d'autres composants (protéines sériques et cellulaires).

Ces milieux sont particulièrement adaptés pour les formes amastigotes de protozoaires tissulaires, tels que les leishmanies cutanées ou cutanéo-muqueuses ou viscérales, ou différents clones de T.cruzi, ou encore Plasmodium ou Babesia.

Comme espèces de leishmanies, on citera L. mexicana, L. amazonensis, et L. braziliensis ou encore L. major, L. guyanensis et L. panamensis.
D'autres milieux de culture préférés, particulièrement appropriés à la culture des formes amastigotes de leishmanies viscérales, comme L. donovani, L. infantum et L. chagasi, comprennent, en outre, des composés soufrés. Il s'agit en particulier, d'acides aminés soufrés, comme la cystéine, et notamment la forme L, et/ou des produits nutritifs comme l'acide bathocuproïne sulfonique.

Pour la différenciation de formes promastigotes en formes amastigotes, dans le cas de leishmanies, le milieu de culture cellulaire représente, par rapport au milieu final, environ 8 à 15 % (V/V), notamment de l'ordre de 10 %, les acides aminés, ou les produits sources de ces acides aminés, comme la trypto caséine de soja ^{R} et la L-glutamine, sont présents, à raison de 4 à 8 % environ (P/V), notamment de 5 à 6 % environ, l'apport en sucre, notamment en glucose, est avantageusement réalisé à raison d'environ 2 à 4 % (P/V), notamment de 2 à 3 %, les agents anti-oxydants comme l'hémine, à raison de 0,0002 à 0,0015 % (P/V) notamment de l'ordre de 0,0005 %, et le glutathion de 0,01 % à 0,05 %, notamment de l'ordre de 0,025 % et la solution de vitamines (100X), à raison de 1 % à 5 % (V/V), notamment de l'ordre de 2 %.

Les composés soufrés, lorsqu'ils sont mis en oeuvre, notamment la L-cystéine sont utilisés à raison d'environ 0,25 à 0,50 % (P/V), notamment de l'ordre de 0, 3 % et l'acide bathocuproïne sulfonique, à raison d'environ 0,004 à 0,008 % (P/V), notamment de l'ordre de 0,005 %.

Pour la culture des stades promastigotes, on utilise avantageusement un milieu de culture élaboré à partir d'un milieu approprié pour la culture de cellules, tel que le milieu RPMI 1640, auquel on ajoute des acides aminés, tels que la L-glutamine et un tampon pour ajuster le pH à une valeur de 7 à 7,5, ce milieu étant également additionné d'un autre milieu approprié pour la culture de cellules, spécialement le milieu 199H M, renfermant des sels inorganiques tels que les sels de Hanks, et des agents anti-oxydants, tels que l'hémine.

Ce milieu est donc dépourvu de tout contaminant sérique et ne contient aucune macromolécule comme prouvé par analyse en gel de polyacrylamide à 10 %.

Le milieu 199H M est utilisé à raison d'environ au moins 2 % (V/V), notamment de 2 à 10 % environ et l'hémine bovine de 0,0001 à 0,0015 % (P/V), notamment de l'ordre de 0,0005 %.

On observera la simplicité de préparation d'un tel milieu, à partir de produits déjà commercialisés. L'absence de sérum conduit de plus avantageusement à un produit de faible coût.

Conformément à l'invention, ces milieux de culture sont mis en oeuvre dans un procédé pour la production massive et continue des formes parasitaires.

Pour l'adaptation et la culture continue in vitro de formes amastigotes, on ensemence des promastigotes de fin de phase exponentielle dans un milieu de culture approprié tel que défini ci-dessus, à raison de 10⁶ à 10⁷ promastigotes/ml de milieu.

Les conditions de réalisation de l'adaptation et de la culture sont avantageusement choisies de manière à assurer de façon reproductible une transformation complète des parasites.

L'adaptation, puis la culture, sont réalisées à des températures de l'ordre de 28 à 36°C, et notamment autour de 32°C à un pH de 5,5 à 6,5. On observe le plus généralement une transformation des formes promastigotes en formes amastigotes dépassant 90 % en 6 à 7 jours. Cette transformation est par exemple totale en quatre jours pour les leishmanies, après un nombre de passages qui varie selon l'espèce étudiée et qui correspond en général à 3 à 9 repiquages, et qui décroît lorsque la température d'incubation augmente.

Pour l'adaptation et la culture continue in vitro de formes promastigotes dites de primo-culture ou de court terme de parasites, tels que les leishmanies, directement obtenues à partir des formes amastigotes, on ensemence des formes amastigotes telles qu'obtenues ci-dessus, à raison de 106 à 10⁷ formes amastigotes/ml de milieu. On utilise avantageusement des formes amastigotes de fin de phase exponentielle. On effectue la culture à une température voisine de l'ambiante mais de préférence n'excédant pas 28°C à un pH de 7 à 7,5 dans un milieu de culture tel que défini plus haut pour le développement de formes promastigotes.

Lorsque l'application visée pour ces stades parasitaires ne nécessite pas le recours à des conditions à la fois axéniques et asériques, sans présence de macromolécules, il est possible dans le cadre de l'invention d'effectuer la culture en milieu seulement axénique, donc en présence de sérum et également en présence de macromolécules. On peut utiliser par exemple du milieu RPMI additionné de sérum de veau foetal.

Les formes promastigotes de court terme ainsi obtenues peuvent être utilisées dans l'étape d'ensemencement évoquée plus haut aux fins de différenciation en amastigotes.

La transformation est très rapide et totale par exemple pour les leishmanies en 4 jours, après 2 à 5 repiquages, selon les espèces.

La mise en oeuvre des dispositions de l'invention permet d'obtenir des cultures standardisées et reproductibles des différentes formes correspondant aux différents stades parasitaires, dépourvues de tout contaminant cellulaire et de macromolécules et capables de se multiplier in vitro.

Les dispositions qui précèdent ont été décrites plus spécialement en rapport avec les formes promastigotes et les formes amastigotes de leishmanies, qu'il s'agisse de leishmanies cutanées, cutanéo-muqueuses, ou encore viscérales, mais s'appliquent également aux stades parasitaires de T. cruzi ou d'autres hématoprotozoaires comme Plasmodium et Babesia.

Ces cultures peuvent être entretenues plusieurs mois, voire plusieurs années pour de nombreuses espèces, en particulier pour les leishmanies, qu'elles soient cutanées, cutanéo-muqueuses ou viscérales, ou pour T.cruzi.

Les stades parasitaires sont capables d'être cultivés à long terme, c'est-à-dire sur plus de 50 passages en cultures in vitro et à court terme, c'est-à-dire récemment transformés de formes promastigotes ou de formes amastigotes (forme antérieure du cycle) inférieur à 10 passages.

L'invention fournit également les moyens pour réaliser un cycle parasitaire in vitro complet. On notera avec avantage que ce cycle complet peut être réalisé en moins de 15 jours.

Un mode de réalisation d'un procédé, selon l'invention, de production des stades correspondant au cycle évolutif d'un parasite tel que les leishmanies est caractérisé en ce qu'il est mis en oeuvre dans les conditions axéniques et asériques définies ci-dessus, en l'absence de macromolécules et qu'il comprend :
- l'ensemencement de formes promastigotes de court terme ou de long terme dans un milieu de culture approprié, comme défini ci-dessus, de manière à obtenir la différenciation en formes amastigotes,
- la récupération des formes amastigotes produites, leur ensemencement et leur culture comme indiqué plus haut de manière à obtenir la différenciation en formes promastigotes,
le cycle étant répété dans sa totalité, ou partiellement, si on le souhaite, et ce, indéfiniment.

Dans un autre mode de réalisation les formes de court terme sont cultivées en conditions seulement axéniques, ou axéniques et asériques en présence de macromolécules.

Par le procédé de l'invention, il est donc possible d'obtenir in vitro les différents stades parasitaires beaucoup plus rapidement et facilement que par les techniques in vivo actuellement utilisées qui mettent en jeu des infections expérimentales qui sont parfois difficilement réalisables. Ces formes parasitaires, amastigotes ou promastigotes, sont exemptes de tout contaminant cellulaire et sont capables de se multiplier in vitro. On dispose ainsi de moyens permettant d'obtenir de manière abondante, voire illimitée, les stades parasitaires, récemment, ou non, différenciés du stade antérieur, notamment de leishmanies et ceux de T. cruzi (épimastigotes, trypomastigotes métacycliques et sanguicoles et amastigotes).

L'invention a également pour objet les formes parasitaires du cycle évolutif d'un protozoaire tissulaire, comme les leishmanies ou T. cruzi, telles qu'obtenues par mise en oeuvre du procédé de culture défini ci-dessus.

Ces formes sont caractérisées en ce que
- elles sont dépourvues de contaminants cellulaires, notamment macrophagiques tissulaires et, plasmatiques accompagnant les formes parasitaires intracellulaires isolées de cultures cellulaires ou de tissus d'animaux expérimentalement infectés, ainsi que de tout contaminant sérique, tout en étant dotées du pouvoir infectieux in vitro et in vivo tel qu'observé sur les formes intracellulaires lorsque ces dernières sont habituellement infectieuses, et des caractéristiques morphologiques, biochimiques et innuno-logiques de celles-ci,
- elles se présentent sous forme d'une population homogène par rapport à l'âge en culture et à l'état de différenciation pour un stade donné du cycle évolutif, cette population, provenant d'une culture standardisée, étant capable de se multiplier in vitro de façon continue.

Les formes amastigotes sont particulièrement préférées étant donné qu'elles correspondent aux formes développées lors de l'infection, chez l'homme ou l'animal.

Ces formes amastigotes sont encore caractérisées en ce qu'elles possèdent une activité enzymatique, plus spécialement une activité peptidasique qualitativement plus complexe que celles des promastigotes, et quantitativement différente, plus particulièrement au niveau des activités cystéine-protéases, comme exposé dans les exemples.

L'invention vise plus spécialement les formes amastigotes de leishmanies aussi bien anthroponotiques, que zoonotiques, ou anthropozoonotiques.

Il s'agit des formes amastigotes de leishmanies cutanées ou cutanéo-muqueuses. Parmi celles-ci, on citera L. mexicana, L. amazonensis, L. braziliensis, L. guyanensis et L. panamensis. Il s'agit encore des amastigotes de leishmanies viscérales comme L. chagasi, L. donovani ou L. infantum.

D'autres formes amastigotes selon l'invention sont celles de différents clones de T. cruzi.

L'invention vise également les formes promastigotes de court terme telles que définies ci-dessus.

Il s'agit de populations directement transformées de formes amastigotes et ayant un pouvoir infectieux analogue à celui de promastigotes récemment isolés d'un hôte infecté.

Chacun des stades parasitaires, promastigote ou amastigote, présente différentes phases de croissance au cours de la multiplication in vitro, à savoir une phase de latence, une phase exponentielle et une phase stationnaire qui correspondent respectivement à la préparation à la division, à une multiplication intense, puis à un stade de non division.

L'invention permet avec avantage d'obtenir de manière ciblée des formes correspondant à l'une de ces phases et d'étudier leurs propriétés et caractéristiques biochimiques spécifiques.

Ces formes sont caractérisées en ce qu'elles sont dépourvues de contaminants cellulaires et sériques, ainsi que de molécules non dialysables au seuil de coupure de 3 kDa.

Il s'agit donc de populations définies correspondant à une phase de leur croissance bien déterminée.

Les formes amastigotes, ou promastigotes de court terme, correspondantes sont particulièrement préférées.

L'invention vise également les extraits polypeptidiques totaux de ces formes parasitaires tels qu'obtenus par lyse des cellules et récupération des produits solubles ou insolubles. Ces extraits sont également appelés dans les exemples extraits antigéniques totaux. Par ces expressions "extraits polypeptidiques totaux" ou "extraits antigéniques totaux", on désigne les produits tels qu'obtenus par lyse des formes parasitaires, qu'ils soient de nature protéique, lipidique ou saccharidique.

Il s'agit en particulier d'extraits polypeptidiques totaux de formes promastigotes de court terme et tout spécialement d'extraits polypeptidiques totaux de formes amastigotes à différentes phases de leur croissance in vitro.

Ces extraits sont caractérisés par leur profil peptidique tel que révélé de manière classique en gel de polyacrylamide SDS-PAGE, en condition réductrice ou non, ou sur gel de polyacrylamide en condition non dénaturante, comme décrit dans les exemples pour certaines espèces de leishmanies.

L'invention vise également les fractions et les déterminants antigéniques, protéines, glucides ou lipides, élués ou isolés à partir de fractions de ces extraits.

Les fractions et déterminants antigéniques de ces extraits reconnus, selon une réaction du type antigène-anticorps, par des sérums d'animaux immunisés avec les extraits polypeptidiques totaux ou des sérums d'infections naturelles ou expérimentales sont particulièrement préférés au regard des applications immunologiques visées par l'invention, et notamment les fractions et déterminants antigéniques spécifiques de base.

Des produits de ce type correspondent aux antigènes exprimés à la surface des formes amastigotes et aux antigènes somatiques présents au niveau de la poche flagellaire ou de type vacuolaire comme mis en évidence par les techniques d'immunofluorescence.

Les molécules purifiées ou semi-purifiées et les solutions enrichies spécifiquement en un ou plusieurs de ces molécules entrent également dans le cadre de l'invention provenant de lyses naturelles.

D'autres produits présentant un grand intérêt au regard des applications biologiques selon l'invention correspondent aux antigènes d'excrétion tels qu'obtenus à partir des surnageants de culture conditionnées par les formes promastigotes ou par les formes amastigotes cultivées dans les conditions axéniques et asériques de l'invention, provenant de lyses naturelles.

Il en est de même des antigènes de différenciation sécrétés durant la différenciation selon le procédé de l'invention des formes promastigotes en formes amastigotes et celle des formes amastigotes en formes promastigotes (au cours de la réalisation du cycle in vitro).

Ces produits antigéniques sont récupérés des surnageants par simple concentration et dialyse. Ces surnageants constituent donc une source purifiée pré-enrichie en produits antigéniques.

L'invention vise également les sérums d'immunisation tels qu'obtenus après administration des extraits, fractions et molécules antigéniques définis ci-dessus selon les techniques habituelles, et les anticorps récupérés à partir de ces sérums.

Elle vise également les sérums d'infection obtenus par infection d'animaux avec les formes amastigotes infectieuses.

Les taux en anticorps de ces sérums varient selon la phase du stade parasitaire et sont plus élevés contre la phase stationnaire des formes amastigotes.

Les anticorps de l'invention sont caractérisés en ce qu'il reconnaissent les peptides spécifiques de formes parasitaires amastigotes ou promastigotes en donnant lieu à une réaction de type antigène-anticorps.

De tels anticorps comprennent ceux ne reconnaissant spécifiquement que les antigènes d'une forme parasitaire, amastigote ou promastigote, appartenant à une espèce homologue, c'est-à-dire à la même espèce que celle utilisée pour leur obtention, la forme reconnue étant celle contre laquelle ils ont été formés.

Il s'agit par exemple des anticorps formés contre une forme amastigote de leishmanie d'une espèce donnée et qui ne reconnaissent spécifiquement que les peptides antigéniques des formes amastigotes de cette espèce de leishmanie.

Les anticorps de l'invention comprennent également ceux qui présentent en outre une faible reconnaissance pour l'autre forme parasitaire de l'espèce considérée.

On citera par exemple les anticorps anti-amastigotes reconnaissant, dans une mesure plus faible, des antigènes des formes promastigotes de la même espèce.

D'autres anticorps encore de l'invention sont en outre capables de reconnaître, mais dans une mesure plus faible, les formes parasitaires d'une espèce hétérologue, ou d'un autre genre comme T. cruzi. Des anticorps de ce type correspondent par exemple à des anticorps anti-amastigotes d'une espèce de leishmanies reconnaissant des peptides amastigotes d'une autre espèce de leishmanies.

Selon un autre aspect, l'invention vise les anticorps monoclonaux tels qu'obtenus avantageusement selon les techniques classiques de fusion d'une lignée cellulaire avec les lymphocytes de la rate ou de ganglions d'un animal immunisé par injection d'un extrait peptidique total, d'une fraction ou d'une molécule antigénique telle que définie ci-dessus par criblage des surnageants des hybridomes obtenus, par exemple selon la technique Elisa ou IFI de manière à révéler les anticorps dirigés spécifiquement contre une forme parasitaire d'une espèce, ainsi que les clones d'hybridomes sécrétant ces anticorps monoclonaux.

Ces anticorps constituent des outils pour séparer ou isoler sélectivement des antigènes spécifiques d'espèces ou de stades à partir d'un milieu les contenant et notamment à partir des extraits polypeptidiques totaux évoqués ci-dessus par des techniques d'immunoaffinité.

La réaction des immunsérums ci-dessus avec des fractions et molécules antigéniques provenant d'une phase donnée du développement de la forme parasitaire permet d'identifier et d'isoler des antigènes spécifiques de ce stade.

La possibilité de produire massivement, grâce à l'invention, des formes amastigotes de culture rend possible l'extraction des ARN totaux et messagers et, à partir de ceux-ci, la réalisation d'une banque d'ADNc.

Selon encore un autre aspect, l'invention vise donc les ARN totaux tels que récupérés à partir de cultures parasitaires d'amastigotes ou de promastigotes, les ARN-m et les ADNc correspondants.

Par comparaison avec une banque d'ADNc de formes promastigotes correspondantes, on met alors en évidence des peptides spécifiques d'un stade parasitaire donné et l'on procède à leur synthèse le cas échéant par génie génétique.

L'obtention conformément à l'invention, de formes amastigotes extracellulaires infectantes in vitro et in vivo, ayant des caractéristiques morphologiques biologiques et biochimiques analogues à celles des formes amastigotes intracellulaires, ouvre de nouvelles et nombreuses applications dans les domaines de la recherche et de l'industrie.

Les formes parasitaires de l'invention sont ainsi particulièrement utiles comme modèles expérimentaux pour effectuer un premier criblage in vitro de produits susceptibles d'être actifs à leur égard in vivo.

Le procécé de criblage de l'invention comprend:
- la mise en contact avec les produits à tester des formes parasitaires, plus spécialement des formes amastigotes telles que cultivées en conditions axénique et notamment asérique, et des formes promastigotes telles que cultivées en milieu complètement défini,
- l'incorporation de nucléotides ou d'acides aminés marqués par un groupe radioactif, par exemple de la thymidine tritiée, pour déterminer l'activité des produits à tester ou la réalisation de tests de viabilité utilisant par exemple un sel de tétrazolium comme le MTT.

Dans l'étape de mise en contact, les formes parasitaires sont utilisées à des concentrations de 106 parasites/ml et l'activité des médicaments est étudiée à des concentrations croissantes.

Les produits à tester peuvent être avantageusement marqués, par exemple par un groupe radio-actif, pour déterminer les mécanismes d'action et le flux des drogues.

L'invention fournit ainsi un modèle permettant de comparer, si on le souhaite, l'activité in vitro de médicaments sur la forme promastigote et sur la forme amastigote à différentes phases de leur croissance d'un parasite donné, et de tester cette activité sur la forme même qui se trouve chez l'hôte.

Elle permet de mieux caractériser l'activité médicamenteuse en mettant en évidence soit un effet lytique (leishmanicide ou trypanocide), soit un effet inhibiteur de la multiplication (leishmaniostatique ou trypanostatique) du produit.

L'utilisation de ces formes parasitaires en tant que modèles expérimentaux permet également d'étudier la chimiorésistance des parasitoses.

On sait en effet que la résistance aux médicaments constituent actuellement un problème important. L'étude des mécanismes conduisant à cette résistance, rendue aisément possible en utilisant les modèles de l'invention, présente donc un grand intérêt.

L'invention vise également un kit pour le criblage de produits susceptibles d'être utilisés pour le traitement de parasitoses, plus spécialement de leishmanies ou de la maladie de Chagas.

Ce kit comprend un support tel qu'une plaque multipuits renfermant les formes parasitaires sur lesquelles on souhaite tester l'activité ou produit à étudier, certaines de ces formes étant utilisées comme témoins, et des réactifs pour déterminer l'activité médicamenteuse du produit sur les formes parasitaires.

Comme indiqué plus haut, les travaux effectués jusqu'à présent n'ont pas permis d'identifier de manière satisfaisante les parasitoses.

Chez l'homme, ou chez les animaux et notamment chez le chien, on établit le plus souvent le diagnostic par exemple de leishmaniose soit en isolant le parasite et en l'identifiant (examen parasitologique), soit en détectant des anticorps (circulants spécifiques) dans le sérum (tests immunosérologiques).

La culture industrielle des formes amastigotes en condition axénique et asérique et en l'absence de macromolécules, permet de disposer d'une source abondante d'outils de diagnostic de grand intérêt.

Ils permettent ainsi de détecter de manière précoce, avec une grande sensibilité et une spécifité élevée, des anticorps circulants dirigés contre les parasites.

L'invention vise donc une méthode de diagnostic d'une parasitose chez l'homme ou l'animal, plus spécialement d'une infection due à des leishmanies ou à T. cruzi, ou de leur détection et identification chez l'insecte vecteur caractérisée en ce qu'elle comprend :
- la mise en contact d'un échantillon biologique provenant du patient ou de l'animal à examiner avec une forme amastigote de culture axénique et asérique ou une forme promastigote de culture asérique comme défini ci-dessus, ou d'un extrait polypeptidique total de ces formes amastigotes ou promastigotes, ou d'un ou plusieurs antigènes spécifiques de cet extrait, purifiés ou semi-purifiés,
- la détection du complexe immunologique.

L'échantillon biologique est plus spécialement un fluide biologique tel que le sang, le sérum ou l'urine.

Lorsqu'on utilise un antigène figuré purifié ou semi-purifié, celui-ci est immobilisé sur un support.

On a recours par exemple à des billes de latex, des plaques Elisa ou des lames de fluorescence.

La révélation de la réaction peut être directe par agglutination macroscopique dans les tests d'agglutination directe ou indirecte en faisant réagir un anticorps conjugué à la fluorescéine (technique d'immunofluorescence indirecte) ou à une enzyme comme la peroxydase ou la phosphatase alcaline (tests ELISA).

Une réaction positive permet donc de diagnostiquer la présence d'anticorps circulants chez le patient ou l'animal examiné.

L'invention vise également un kit pour la mise en oeuvre d'une méthode de diagnostic d'une parasitose, comme défini ci-dessus, chez l'homme ou l'animal.

Ce kit est caractérisé en ce qu'il comprend :
- les réactifs antigéniques sous forme immobilisée, à savoir les formes amastigotes de culture axénique ou de culture axénique et asérique ou les formes promastigotes de culture définie, les extraits polypeptidiques totaux obtenus à partir de ces formes ou les antigènes spécifiques de ces extraits avec, le cas échéant,
- un témoin positif, constitué par un sérum de titre connu,
- un témoin négatif, ainsi que
- les tampons et réactifs utilisables pour la révélation de la réaction immunologique.

La détection des anticorps circulants anti-parasites chez un patient ou un animal peut être réalisée en mettant en compétition avec les anticorps de l'échantillon, un anticorps spécifique de l'antigène, en particulier un anticorps monoclonal. Cet anticorps comporte avantageusement un marqueur, par exemple un groupe radioactif ou enzymatique.

En variante, la méthode de diagnostic est basée sur la mise en évidence de la présence des déterminants antigéniques des formes parasitaires (détection des antigènes circulants).

Dans cette variante, l'échantillon biologique provenant de l'homme ou de l'animal est mis en contact avec des anticorps spécifiques dirigés contre les antigènes de la forme parasitaire, ou des fragments de ces anticorps.

La détection de la réaction immunologique est réalisée par exemple en utilisant le même anticorps mais marqué.

On notera avec intérêt que la possibilité fournie par l'invention de détecter des antigènes circulants, c'est-à-dire ceux qui apparaissent rapidement chez le sujet infecté, permet d'effectuer un diagnostic précoce de la maladie.

On utilise les anticorps dirigés contre les formes promastigotes ou amastigotes, en particulier des anticorps monoclonaux, ces formes provenant de parasites de différentes espèces de leishmanies comme L. infantum ou T. cruzi.

Un kit de diagnostic correspondant comprend :
- une phase solide appropriée servant de support pour la réaction immunologique, telle qu'une plaque de micro-titration,
- une préparation d'anticorps selon l'invention tels que définis ci-dessus, ou de fragments de ces derniers, immobilisés sur un support,
- un témoin positif, constitué par un sérum de titre connu,
- un témoin négatif, ainsi que
- les tampons et réactifs utilisables pour la réalisation de la réaction immunologique et notamment l'anticorps homologue marqué.

Selon un autre aspect, les outils de diagnostic de l'invention permettent d'effectuer un diagnostic différentiel entre plusieurs parasitoses.

On observe en effet aussi bien chez l'homme que chez l'animal, par exemple les rongeurs, des réactions croisées entre T. cruzi, T. rangeli (trypanosome non pathogène chez l'homme) et des leishmanies viscérales ou cutanées.

L'étude des formes parasitaires de l'invention, des extraits polypeptidiques totaux et des antigènes spécifiques définis plus haut a mis en évidence leurs fortes propriétés immunogéniques.

L'invention vise donc également leur utilisation en tant qu'agents protecteurs vis-à-vis de parasitoses, plus spécialement de leishmanioses et de la maladie de Chagas.

Les compositions de vaccins de l'invention sont caractérisées en ce qu'elles sont élaborées à partir des formes amastigotes ou de formes promastigotes de culture axénique et asérique, en l'absence de macromolécules, comme défini ci-dessus, à différentes phases de leur croissance, ou de leurs constituants, en association avec un véhicule et/ou un adjuvant d'administration.

Les constituants des formes amastigotes ou promastigotes en question comprennent les extraits polypeptidiques totaux obtenus par lyse de ces formes parasitaires. Ils comprennent également les fractions antigéniques et les antigènes protecteurs spécifiques isolés à partir des formes parasitaires, mais aussi à partir des surnageants de cultures conditionnés par les parasites lorsqu'ils sont cultivés en milieux complètement définis.

L'administration de ces agents de protection à l'homme ou l'animal permet de leur conférer une immunité globale contre les parasitoses dans lesquelles ils interviennent dans le processus d'infection naturel.

Leur effet avantageux a été mis en évidence spécialement au niveau de la réponse immunitaire à médiation cellulaire favorisant la stimulation des lymphocytes T sécrétant l'interleukine 2, et l'interféron gamma (TH₁) ou inhibant l'activation des cellules T sécrétant les interleukines 4 et 5 (TH₂).

Ces agents de protection sont avantageusement sous forme lyophilisée.

Dans le cas d'extraits polypeptidiques totaux, les compositions de vaccins sont administrées par la voie sous-cutanée à raison de 100 à 1000 µg chez l'homme et de 100 à 500 µg chez le chien en présence d'adjuvants tel que le muramyldipeptide ou la saponine ou en présence de cytokine comme l'interféron gamma.

Les antigènes d'excrétion-sécrétion des surnageants de culture métabolisés par les formes amastigotes de l'invention offrent une stratégie originale dans le développement de vaccins contre les maladies parasitaires.

On soulignera en particulier leur intérêt pour élaborer des vaccins contre les leishmanioses viscérales, canines ou humaines (L. infantum et L. chagasi). Ils correspondent en effet avantageusement aux formes présentes chez l'hôte infecté.

Les différentes expérimentations effectuées ont permis de mettre en évidence leurs propriétés immunogènes et leur effet protecteur chez l'homme ou l'animal.

Pour préparer des vaccins à partir desdits antigènes, ou exoantigènes, on utilise les surnageants concentrés dialysés de cultures d'amastigotes, ou les cultures elles-mêmes contenant les parasites et les surnageants, les parasites étant tués par exemple par traitement thermique, ou des extraits de ces solutions.

Ces produits sont utilisés avec des adjuvants comme le MDP ou des cytokines, comme l'interféron gamma.

On a recours à un protocole d'immunisation à long terme ou à court terme. Le protocole à long terme est par exemple réalisé par injection toutes les trois semaines de la préparation vaccinale, aux jours j = 0, j = 21 et j = 42. Pour un protocole à court terme, on réalise par exemple deux injections à 15 jours d'intervalle.

Avant l'épreuve virulente, pour l'animal, on vérifie que l'immunité d'hypersensibilité à médiation cellulaire a bien été induite vers l'activation des cellules TH1 sécrétant l'interleukine 2 et l'interféron gamma.

Ces analyses peuvent être également réalisées chez l'homme au terme de l'immunisation.

On rapporte dans les exemples qui suivent d'autres caractéristiques et avantages de l'invention.

Dans ces exemples, il est fait référence aux figures 1 à 13, qui représentent respectivement :
- la figure 1a et 1b, les courbes de différenciation des formes promastigotes en formes amastigotes et de croissance des parasites pour L. amazonensis,
- la figure 2, le pourcentage de formes amastigotes de L. mexicana à différentes températures de culture,
- les figures 3a et 3b, les cinétiques de culture des formes amastigotes de différentes leishmanies cutanées et cutanéo-muqueuses (3a) et d'une leishmanie viscérale (3b).
- les figures 4a à 4c, les cinétiques de culture de formes promastigotes de leishmanies cutanées et cutanéo-muqueuses de leishmanies viscérales et de T. cruzi,
- la figure 5 a, les caractéristiques cytologiques et la figure 5 b les caractéristiques ultrastructurales des formes amastigotes de culture de leishmanies,
- les figures 6a et 6b, les analyses électrophorétiques en gel de polyacrylamide SDS-PAGE des profils polypeptidiques totaux de formes amastigotes de lésion et de culture selon l'invention, et de formes promastigotes de différentes leishmanies,
- les figures 6c et 6d, les analyses électrophorétiques en gel d'empreinte des activités protéasiques de ces formes,
- les figures 7a à 7d, les analyses indiquées ci-dessus, mais concernant des leishmanies viscérales,
- les figures 8a et 8b, l'analyse des profils antigéniques de leishmanies par la technique d'immunoblotting à l'aide de sérums d'immunisation de lapin homologue,
- les figures 9 et 10, les cinétiques de différenciation de formes amastigotes obtenues selon l'invention en formes promastigotes, respectivement, pour des leishmanies cutanées et cutanéo-muqueuses, et pour des leishmanies viscérales,
- les figures 11 et 12, les cinétiques d'incorporation de [³⁵S] méthionine par les protéines totales ou d'excrétion-sécrétion de L. infantum et L. amazonensis, respectivement, et
- la figure 13, le degré de résistance à la pentamidine de formes parasitaires de L. mexicana

Les exemples 1 à 3 se rapportent à des milieux de culture axéniques, ou axéniques et asériques, mais contenant des macromolécules. Ces milieux sont décrits à toutes fins utiles dès lors qu'ils sont utilisables lors de l'adaptation d'une forme parasitaire dans un milieu donné et sont remplacés ensuite avantageusement, au cours des passages successifs, par des milieux axéniques et asériques, dépourvus de molécules non dialysables à un seuil de coupure de 3 kDa. Selon l'application envisagée pour les formes parasitaires, ils peuvent être mis en oeuvre dans l'une ou plusieurs étapes de réalisation du cycle évolutif d'une forme parasitaire.

### Exemple 1 : Elaboration d'un milieu de culture acellulaire pour formes amastigotes de leishmanies cutanées ou cutanéo-muqueuses ou de T. cruzi.

On rapporte dans le tableau 1 suivant la formulation d'un milieu de culture supportant la croissance des formes amastigotes en conditions axénique de différentes espèces de leishmanies responsables de leishmanioses cutanées et cutanéo-muqueuses et de différentes souches de T. cruzi.

**Tableau 1**

| Constituants | Quantités pour 1 litre |
|---|---|
| Milieu de base | |
| - Milieu 199H R (x10) (avec sels de Hanks)* | 100 ml |
| - Trypto-caséine de soja^{R} | 5 g |
| - NaHCO₃ | 0,35 g |
| - L-glutamine | 0,75 g |
| - HEPES | 5,95 g |
| - D(+) glucose | 2,50 g |
| - H₂O | Q.S. 800 ml |
| additifs | |
| - Hémine bovine | 0,015 g |
| - Sérum de veau foetal | 200 ml |

| | |
|---|---|
| * milieu 199 H commercialisé par Gibco BRL, réf 042-01181 du catalogue de 1994. | |

Ce milieu, appelé dans la suite des exemples M1, est préparé comme suit :

A 100 ml de milieu 199H (avec sels de Hanks), concentré 10 fois, sont additionnés successivement dans les proportions indiquées dans le tableau, la trypto caséine de soja^{R}, le bicarbonate de sodium, la L-glutamine, l'HEPES et le D(+) glucose. Le volume du milieu est alors ajusté à 800 ml avec de l'eau désionisée-distillée. Ce milieu de base est conservé pendant plusieurs mois à -20°C. Lors de son utilisation, l'hémine bovine (0,023 mM) et le sérum de veau foetal (200 ml) préalablement décomplémenté (56°C pendant 30 minutes) sont ajoutés aux 800 ml de milieu de base. Le milieu M1 résultant, conservé à 4°C, peut être utilisé durant 3 semaines sans altération de ses propriétés. Le pH du milieu est de 6,5 ± 0,1 et la valeur de l'osmolarité a été déterminée à 443,3 ± 2,3 milli osmoles par kg d'eau.

On rappelle que les sels de Hanks répondent à la composition suivante : CaCl₂, 2H₂O (1,8 g/l), KCl (4 g/l), KH₂PO₄ (0,6 g/l), MgSO₄, 7H₂O (2 g/l), NaCl (80 g/l) et Na HPO₄, 7H₂O, (0,48 g/l). Les concentrations indiquées correspondent au milieu 199H 10X.

### Exemple 2 : Elaboration d'un milieu de culture acellulaire pour formes amastigotes de leishmanies viscérales.

On additionne au milieu M1 décrit ci-dessus de la L-cystéine (3 mM) et de l'acide bathocuproine disulfonique (0,01 mM). La composition du milieu obtenu, appelé ci-après milieu M2, est donnée dans le tableau 2 suivant :

**Tableau 2**

| **Constituants** | **Quantités** |
|---|---|
| - Milieu M1 | 1000 ml |
| - L-cystéine | 3 mM |
| - acide bathocuproïne disulfonique | 0,01 mM |

### Exemple 3 : Elaboration de milieux de culture axéniques et asériques pour formes amastigotes de leishmanies.

Des milieux complètement définis supportant la croissance des formes amastigotes de leishmanies en conditions axénique et asérique ont été mis au point. Pour les formes amastigotes, le sérum de veau foetal du milieu M1 ou M2 est remplacé par un complexe albumine de sérum bovin (BSA) - acide linoléique à raison de 20 µg d'acide linoléique par ml de milieu M1 ou M2. Le pH du milieu est de 6,5 ± 0,1 et la valeur de l'osmolarité a été déterminée à 467 ± 2,9 milliosmoles par kg d'eau.

On rapporte dans le tableau 3 ci-après la composition correspondante du milieu obtenu, appelé M3.

**Tableau 3**

| **Constituants** | **Quantités** |
|---|---|
| - M1 ou M2 sans SVF | 100 ml |
| - BSA - acide linoléique | 0,002 % |
| | (P acide linoléique /V) |

### Exemple 4 : Elaboration d'un milieu de culture acellulaire et asérique, dépourvu de macromolécules (composé de molécules dialysables à un seuil de coupure de 3 kDa) pour formes amastigotes.

On rapporte dans le tableau 4 la formulation d'un milieu selon l'invention pour la différenciation et la culture de formes amastigotes de leishmanies cutanées, cutanéo-muqueuses et viscérales et de différentes souches de T. cruzi.

**Tableau 4**

| Constituants | Quantités pour environ 800 ml |
|---|---|
| Milieu de base | |
| - Milieu 199H ^{R} (x10) (avec sels de Hanks)* | 100 ml |
| - Trypto-caséine de soja^{R} | 5 g |
| - NaHCO₃ | 0,35 g |
| - L-glutamine | 0,75 g |
| - HEPES | 5,95 g |
| - D(+) glucose | 2,50 g |
| - H₂O | Q.S. 800 ml |
| - Milieu 199 H modifié^{R} (x 10)** | 4 ml (5 %) |
| additifs | |
| - Hémine bovine | 0,009 mM |
| - glutathion réduit | 0,08 mM |
| - solution de vitamines (X 100) | 2 % |

| | |
|---|---|
| * milieu 199 H commercialisé par Gibco BRL, réf 042-01181 du catalogue de 1992 | |
| ** milieu 199 H modifié commercialisé par Flow Laboratoires, réf 14230-54 du catalogue de 1992. | |

Ce milieu, appelé dans la suite des exemples MA1, est préparé comme suit :

On élabore tout d'abord le milieu de base suivant :

A 100 ml de milieu 199H (avec sels de Hanks), concentré 10 fois (ou 10 x), tel que commercialisé par Gibco BRL, sont additionnés successivement dans les proportions indiquées dans le tableau, la trypto caséine de soja^{R}, le bicarbonate de sodium, la L-glutamine, l'HEPES et le D(+) glucose. Le volume du milieu est alors ajusté à 800 ml avec de l'eau désionisée-distillée. On ajoute alors du milieu 199 H modifié concentré dix fois, tel que commercialisé par Flow Laboratories, préalablement chauffé à 56°C pendant 45 minutes.

Ce milieu de base est conservé pendant plusieurs mois à -20°C. Lors de son utilisation, on ajoute aux 800 ml de milieu de base l'hémine bovine (0,009 mM), le glutathion réduit (0,08 mM) tel que commercialisé par Boehringer Mannheim sous la référence 127744 et une solution de vitamines (2 %), concentrée 100 fois, telle que celle commercialisée par Gibco BRL, réf 12414-017. Cette solution comporte les vitamines suivantes avec les concentrations indiquées entre parenthèses: biotine (0,4 mg/L), pantothénate de calcium D (0,5 mg/L), chlorure de choline (6 mg/L), acide folique (2 mg/L), i-inositol (70 mg), nicotinamide (2 mg/L), acide para-aminobenzoïque (2 mg/L), pyridoxine-HCl (2 mg), riboflavine (0,4 mg/L), thiamine-HCl (2 mg/L) et vitamine B12 (0,01).

Le milieu MA1 résultant, conservé à 4°C, peut être utilisé durant 3 semaines sans altération de ses propriétés. Le pH du milieu est de 6,5 ± 0,1 et la valeur de l'osmolarité a été déterminée à 412,3 ± 3,1 milli osmoles par kg d'eau.

### Exemple 5 : Elaboration d'un milieu de culture selon l'exemple 4 approprié pour des formes amastigotes de leishmanies viscérales.

On additionne si nécessaire à 1000 ml du milieu MA1 de l'exemple 4 de la L-cystéine (3 mM) et de l'acide bathocuproïne disulfonique (0,01 mM)

### Exemple 6 : Elaboration de Milieux de culture axéniques et asériques, dépourvus de macromolécules pour les formes promastigotes.

Le milieu asérique conçu pour la culture des formes promastigotes est constitué d'un mélange de milieux de culture commercialisés. A un litre de milieu RPMI 1640 concentré 1,1 fois et contenant 5,95 g d'Hepès, on ajoute 20 ml de milieu 199H (avec sels de Hanks) modifié et 0,5 mg d'hémine bovine. Ce milieu est conservé 15 jours à +4°C sans altération de ses propriétés. Le pH du milieu est de 7,2 ± 0,1 et la valeur de l'osmolarité a été déterminée à 353 ± 3 milliosmoles par kg d'eau.

La formulation pour un litre du milieu de culture supportant la croissance des formes promastigotes de leishmanies en condition asérique est rapportée dans le tableau 5 ci-après et est appelé MP.

**Tableau 5**

| Constituants | Quantités |
|---|---|
| RPMI 1640 (1,1 X) | 1000 ml |
| avec L-glutamine et HEPES Milieu 199H modifié* (10 X) | 2 % (P/V) |
| Hémine bovine | 0,0005 % (P/V) |

| | |
|---|---|
| * milieu 199 H de Gibco BRL cité plus haut | |

### Exemple 7 : Adaptation et culture de formes amastigotes de leishmanies en condition axénique.

Les formes promastigotes de différentes espèces de leishmanies sont mises en culture, puis sont différenciées en formes amastigotes.

On indique ci-après les résultats de travaux effectués sur 19 souches de leishmanies.

### a. souches de leishmanies

Les principales caractéristiques (pays, hôtes, année d'isolement et espèces) sont résumées dans le tableau 6 suivant. La caractérisation subspécifique des différentes espèces de leishmanies a été réalisée par typification génétique en analysant plus de 10 loci isoenzymatiques variables.

**Tableau 6**

| Désignation & Source | Espèces | Nombre de passages | Nombre amastigotes (x10⁷/ml) |
|---|---|---|---|
| MHOM/BR/79/LI-01 | L. chagasi | 75 | 7,2 |
| MHOM/MA/67/JTMAP | L. infantum | 39 | 7,3 |
| Clone 1 263 | | | |
| MHOM/MA/27/JTMAP | L. infantum | 48 | 6,9 |
| Clone 7 263 | | | |
| MHOM/IN(--)/61/L-13 | L. donovani | 64 | 5,9 |
| | | | |
| MHOM/--/--/IT-2217 | L. donovani | 56 | 6,1 |
| MHOM/IN/80/Ldd 8 Cl₂ | L. donovani | 44 | 5,9 |
| MHOM/IN/80/Ldd 8 Cl₂ | L. donovani | 41 | 4,8 |
| R60 | | | |
| MNYC/BZ/62/M-379 | L. mexicana | 167 | 7,5 |
| MHOM/BO/83/LPZ-155 | L. mexicana | 97 | 6,2 |
| MHOM/BR/76/LTB-012 | L. amazonensis | 149 | 7,2 |
| MHOM/BR/73/M-2269 | L. amazonensis | 89 | 5,1 |
| MHOM/BR/75/M-2904 | L. braziliensis | 145 | 6,3 |
| MHOM/BO/90/CS | L. braziliensis | 64 | 6,2 |
| MHOM/BO/90/AN | L. braziliensis | 37 | 5,9 |
| MHOM/BR/75/M-4147 | L. guyanensis | 94 | 6,6 |
| MHOM/BR/78/M-5378 | L. guyanensis | 42 | 5,4 |
| MCHO/PA/00/M-4039 | L. panamensis | 85 | 5,9 |
| MHOM/PA/71/LS-94 | L. panamensis | 74 | 5,2 |
| MHOM/EQ/91/A-8044 | L. panamensis | 39 | 4,8 |

### b. culture des formes promastigotes

Les formes promastigotes de ces différentes souches de leishmanies sont cultivées aux fins d'adaptation à 26 ± 1°C dans un milieu synthétique monophasique liquide, le RPMI 1640, additionné de 10 % de sérum de veau foetal (en abrégé SVF) préalablement décomplémenté à 56°C pendant 30 minutes.

La composition du milieu est la suivante :
- RPMI 1640 10,40 g
- HEPES 5,95 g
- NaHCO₃ 2,20 g
- H₂O 900,00 ml

Le pH est ajusté à 7,2 avec de la soude 1N ; le milieu est stérilisé par filtration sur une membrane Millipore ^{R} de 0,22 µm de porosité et peut être conservé ainsi pendant un mois à 4°C. Lors de son utilisation, 100 ml de SVF décomplémenté sont ajoutés.

Les souches de leishmanies sont maintenues en culture routinière par deux repiquages hebdomadaires qui consistent à ensemencer des formes promastigotes de phase exponentielle (5 x 105 parasites par ml) dans 5 ml de milieu en flacon de culture de 50 ml. Les différentes souches sont conservées par cryocongélation à -180°C en présence de 5 % de DMSO.

### c. Différenciation in vitro des formes promastigotes en formes amastigotes :

5 x 10⁷ formes promastigotes, de fin de phase exponentielle, de leishmanies, obtenues comme indiqué ci-dessus, sont ensemencées dans 10 ml du milieu de culture M1 pour les promastigotes de leishmanies cutanées ou cutanéo-muqueuses et du milieu M2 pour celles de leishmanies viscérales.

Aux différents jours de la cinétique de différenciation, 20 µl de la suspension parasitaire sont déposés sur une lame, séchés rapidement puis fixés au méthanol absolu et colorés selon la méthode de Giemsa. Le pourcentage de formes amastigotes est déterminé par comptage du nombre de formes amastigotes par rapport au nombre total de parasites en microscopie photonique (x 400) dans 20 champs successifs.

Ces cultures sont réalisées à une température comprise entre 28 et 36°C.

En opérant comme indiqué ci-dessus, on a réalisé des essais en dupliquette à 28, 32, 34 et 36°C avec L.mexicana et L.amazonensis et 28 et 32°C avec L.braziliensis, L. guyanensis et L. panamensis.

Les différentes courbes de différenciation obtenues en fonction de la température, lors d'un premier passage en milieu M1 montrent que, d'une manière générale, dans les conditions de ces expériences,
- la croissance in vitro (nombre de parasites par ml) des organismes étudiés ne semble pas réellement altérée par l'augmentation de la température d'incubation de la culture. Seule la cinétique de multiplication de L.amazonensis à 36°C est fortement ralentie ;
- la vitesse de différenciation (% de formes amastigotes au cours du temps) ainsi que les pourcentages de formes amastigotes, déterminés au huitième jour de la culture, augmentent significativement et de façon corrélée avec l'élévation de la température d'incubation;
- plus de 90 % des formes promastigotes sont transformées en formes amastigotes au terme des différentes cinétiques de différenciation et ceci, pour des températures d'incubation supérieures ou égales à 32°C.

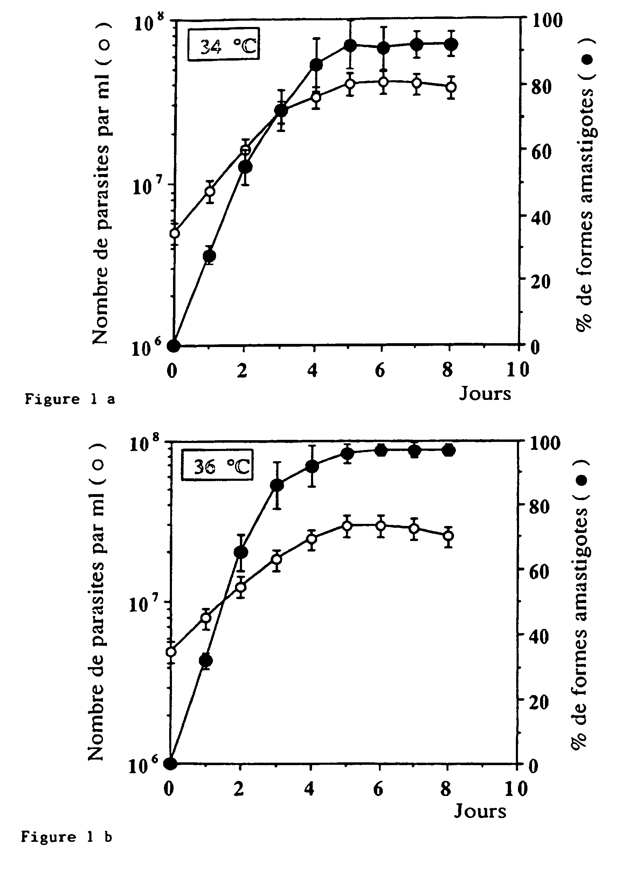

Afin d'illustrer les résultats obtenus, on a rapporté, sur les figures 1a et 1b, le nombre de parasites par ml (0) et le pourcentage de formes amastigotes (●) en fonction du temps (en jours) pour L.mexicana à 34 et 36°C. On constate une différenciation élevée en formes amastigotes, qui augmente avec la température.

Dans les mêmes conditions, mais en utilisant un milieu M2, on a étudié la différenciation de leishmanies viscérales comme L. chagasi et L. donovani. On obtient des pourcentages analogues de formes amastigotes.

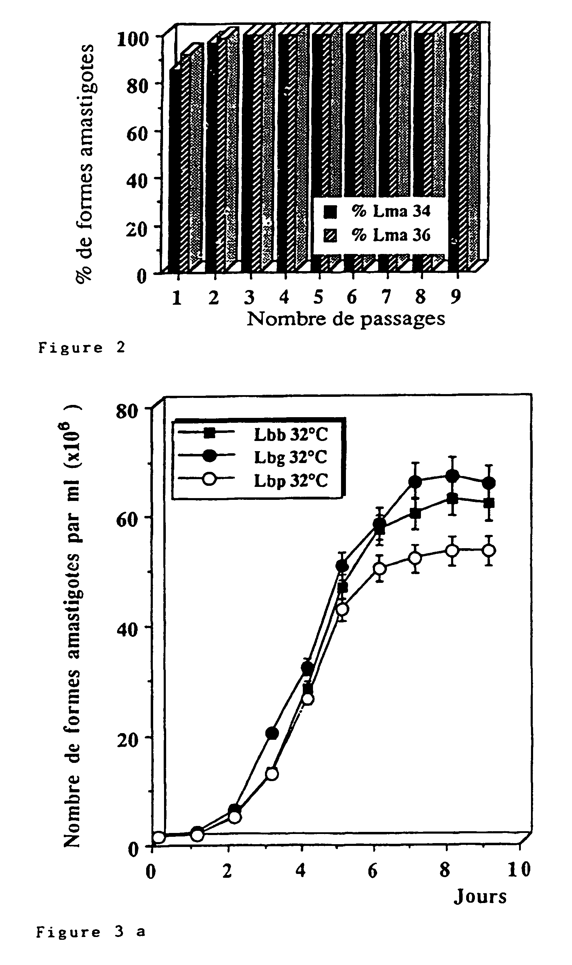

On notera que pour des températures supérieures ou égales à 32°C, la transformation des formes promastigotes en formes amastigotes est totale en 4 jours après un nombre de passages qui varie selon les espèces de leishmanies étudiées et qui décroît lorsque la température d'incubation s'élève. A 32°C, 3 ou 4, 6 à 8 et 8 ou 9 repiquages sont respectivement nécessaires pour les leishmanioses cutanées, cutanéo-muqueuses et viscérales, alors que 2 ou 3 et 7 passages à 36°C pour les leishmanioses cutanées et viscérales étudiées comme représenté sur la figure 2 qui donne le pourcentage de formes amastigotes en fonction du nombre de passages pour L. mexicana à 34°C et à 36°C.

### d. Cinétique de croissance des formes amastigotes

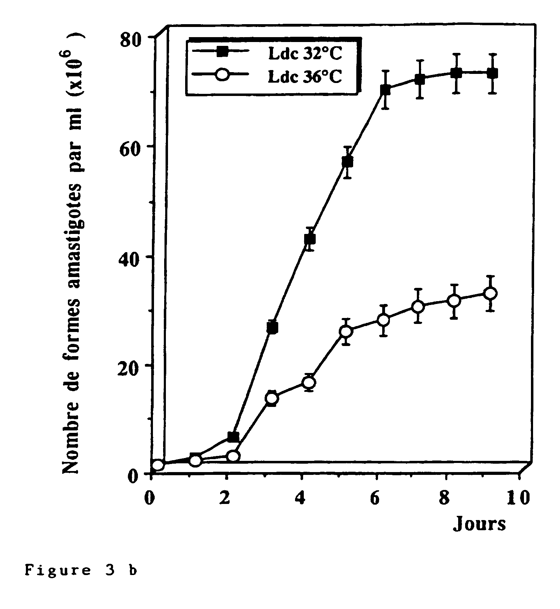

Des courbes de cinétique de culture des formes amastigotes de différentes espèces de leishmanies ont été établies à différentes températures. On rapporte sur les figures 3a et 3b les résultats obtenus après au moins 25 repiquages de 5 x 10⁶ formes amastigotes dans 10 ml de milieu M1, à 32°C pour L. braziliensis (●), L. guyanensis (-●-) et L. panamensis (-○-) et à 32°C et 36°C pour L. chagasi.

Les trois phases caractéristiques du cycle de croissance cellulaire observées classiquement au cours de la culture des formes promastigotes sont retrouvées :
- une phase de latence (2 jours),
- une phase exponentielle pendant laquelle les parasites se multiplient rapidement (4 à 5 jours selon les espèces de leishmanies),
- une phase stationnaire qui correspond à un stade de non division (à partir du 6ème ou 7ème jour).

On observe une vitesse de multiplication, calculée au cours de la phase exponentielle (correspondant au temps de doublement réél), sensiblement la même pour les différentes cinétiques de culture, mais le temps de doublement des formes amastigotes est significativement différent. Il varie de 27 à 32 heures selon les espèces étudiées (alors qu'il n'est que de 16 à 21 heures pour les formes promastigotes correspondantes dans les mêmes conditions d'inoculum, du volume de milieu et d'aération). Enfin, les concentrations parasitaires déterminées à la confluence des cultures de formes amastigotes de leishmanies varient dans les conditions de ces expériences, de 4,8 à 7,5 x 10⁷ formes amastigotes par ml de culture en 6 ou 7 jours selon l'espèce étudiée (voir tableau 5).

Il est possible de cultiver les formes amastigotes des leishmanioses viscérales à 34°C et même 36°C. Les courbes de croissance sont semblables à celles obtenues à 32°C.

### Exemple 8 :

Environ 10⁷ formes amastigotes de fin de phase exponentielle des principales espèces de leishmanies, cultivées en milieu M3 sont encemencées dans 5 ml de milieu MA1 à différentes températures. Des essais en tripliquette à 32°C ont été réalisés avec L. mexicana et L. amazonensis et L. donovani et à 36°C pour L. infantum.

Si les vitamines sont omises dans le milieu, les formes amastigotes sont lysées au bout du 5ème repiquage. En absence de glutathion réduit les cultures persistent pendant 15 passages. Le milieu MA1 supporte la croissance continue (supérieur à 40 passages) des formes amastigotes en conditions axénique et asérique des différentes espèces de leishmanioses cutanées et cutanéo-muqueuses et viscérales.

Les courbes de cinétique de culture des formes amastigotes des différentes espèces de leishmanies ont été établies aux différentes températures après au moins 10 repiquages de 5 x 10⁵ formes amastigotes par ml dans 5 ml de milieu MA1. D'une manière générale, elles présentent une bonne analogie avec celles obtenues en milieu sérique M3.

Des cultures massives, permettant une production importante de formes amastigotes, ont aussi été réalisées dans des flacons de culture de 600 ml (contenant 200 à 300 ml de volume utile) après un passage intermédiaire en boite de 200 ml (contenant 50 à 75 ml de volume utile). D'une manière générale, environ 10⁹ parasites lavés trois fois sont obtenus pour 75 ml de culture.

D'une manière générale, les formes amastigotes cultivées en condition axénique et asérique, et en l'absence de macromolécules conservent leur pouvoir infectieux in vitro et in vivo. Les profils polypeptidiques et protéasiques sont analogues à ceux présentés par les formes amastigotes cultivées en condition axénique (voir les résultats donnés pour L. amazonensis et L. infantum).

On rapporte dans le tableau 7, les principales caractéristiques des souches de leishmanies cultivées en conditions axénique et asérique, sans macromolécules, sous forme amastigote.

**Tableau 7**

| Désignation et source | espèce | nombre de passages | croissance moyenne (x 107/ml) |
|---|---|---|---|
| MHOM/MA(BE)67/IT-263 | L. infantum | 68 | 6,1 |
| MHOM/MA/67/IT-263 | L. infantum | 42 | 6,4 |
| clone 2 | | | |
| MHOM/MA/67/IT-263 | L. infantum | 49 | 7,0 |
| clone 7 | | | |
| MHOM/../../IT-2217 | L. donovani | 49 | 7,3 |
| MHOM/IN/80/DD8 | L. donovani | 26 | 6,5 |
| clone 2 | | | |
| MHYC/BZ/62/M-379 | L. mexicana | 72 | 7,6 |
| MHOM/VE/76/JAP-78 | L. amazonensis | 76 | 6,9 |
| MHOM/BR/76/LTB-012 | L. amazonensis | 42 | 6,9 |
| MHOM/BR/73/M-2269 | L. amazonensis | 85 | 5,9 |
| MHOM/BO/90/CS | L. braziliensis | 16 | 5,8 |

### Exemple 9 : Adaptation et culture continue des formes promastigotes en milieux complètement définis.

On indique ci-après les résultats de travaux effectués sur 25 souches de leishmanies et une souche de T. cruzi.

Les principales caractéristiques (pays, hôtes, année d'isolement et espèces) de ces souches sont résumées dans le tableau 8 suivant. La caractérisation subspécifique des différentes espèces de leishmanies et de T. cruzi a été réalisée par typification génétique en analysant plus de 10 loci isoenzymatiques variables.

**Tableau 8**

| Désignation et Source | Espèces | Nombre de passages | Nombre de promastigotes (x10⁷/ml) |
|---|---|---|---|
| MHOM/BR/79/LI-01 | L. chagasi | 249 | 6,6 |
| MHOM/MA(BE)/67/IT-263 | L. infantum | 89 | 5,6 |
| MHOM/MA/67/IT-263 clone 2 | L. infantum | 59 | 6,5 |
| MHOM/MA/67/IT-263 clone 7 | L. infantum | 61 | 7,1 |
| MHOM/IN/83H 570 | L. donovani | 161 | 6,9 |
| MHOM/IN/(--)/61/L-13 | L. donovani | 149 | 7,8 |
| MHOM/../../IT-2217 | L. donovani | 126 | 7,2 |
| MHOM/IN/80/DD8 clone 2 | L. donovani | 129 | 7,3 |
| MHYC/BZ/62/M-379 | L. mexicana | 229 | 7,9 |
| MHOM/BO/83/LPZ-155 | L. mexicana | 102 | 5,6 |
| MHOM/VE/76/JAP-78 | L. amazonensis | 176 | 6,5 |
| MHOM/BR/76/LTB-012 | L. amazonensis | 142 | 6,9 |
| MHOM/BR/73/M-2269 | L. amazonensis | 85 | 7,7 |
| MHOM/BR/72/1670 | L. braziliensis | 111 | 6,5 |
| MHOM/BR/75/M-2904 | L. braziliensis | 99 | 7,1 |
| MHOM/BO/90/CS | L. braziliensis | 86 | 5,8 |
| MHOM/BO/90/AN | L. braziliensis | 59 | 6,1 |
| MCHO/PA/OO/M-4039 | L. panamensis | 107 | 6,6 |
| MHOM/PA/71/LS-94 | L. panamensis | 136 | 5,3 |
| MHOM/91/EQ/A8044 | L. panamensis | 98 | 6,8 |
| MHOM/BR/78/M-5378 | L. guyanensis | 76 | 5,3 |
| MHOM/BR/75/M-4147 | L. guyanensis | 113 | 6,2 |
| MHOM/PE/85/FR-6 | L. peruviana | 79 | 5,5 |
| schnur strain | L. major | 85 | 5,0 |
| LTD | L. tropica | 80 | 6,8 |
| SOUCHE TEHUANTEPEC | T. cruzi | 36 | 5,1 |

### a. Culture des formes promastigotes en milieux complètement définis.

Les formes promastigotes des différentes souches de leishmanies sont cultivées classiquement à 26°C dans le milieu RPMI 1640 additionné de 10 % de sérum de veau foetal (préalablement décomplémenté) comme il est indiqué dans l'exemple 4, b ci-dessus. La souche de T. cruzi est cultivée dans le même milieu, mais à une température d'incubation de 28°C.

Une adaptation progressive des formes promastigotes cultivées classiquement dans le milieu RPMI 1640 contenant 10 % de sérum de veau foetal, est réalisée dans le milieu M4. Tous les essais sont réalisés en dupliquette à 26 ± 1°C pour les leishmanies et 28 ± 1°C pour T. cruzi en volume de 5 ml de milieu dans des flacons de 25 cm2 (50 ml). Les cultures sont tout d'abord diluées au demi dans le milieu M4 pendant au moins 2 passages qui ont lieu tous les 4 à 5 jours, puis au 1/5, au 1/10 et au 1/20 pendant un nombre de passages qui varie, selon les espèces étudiées, de 2 à 7 pour chaque nouvelle concentration pour enfin réaliser des repiquages correspondant à un ensemencement de 5 X 10⁵ parasites par ml de milieu M4.

### b. Cinétique de croissance des formes promastigotes dans le milieu M4

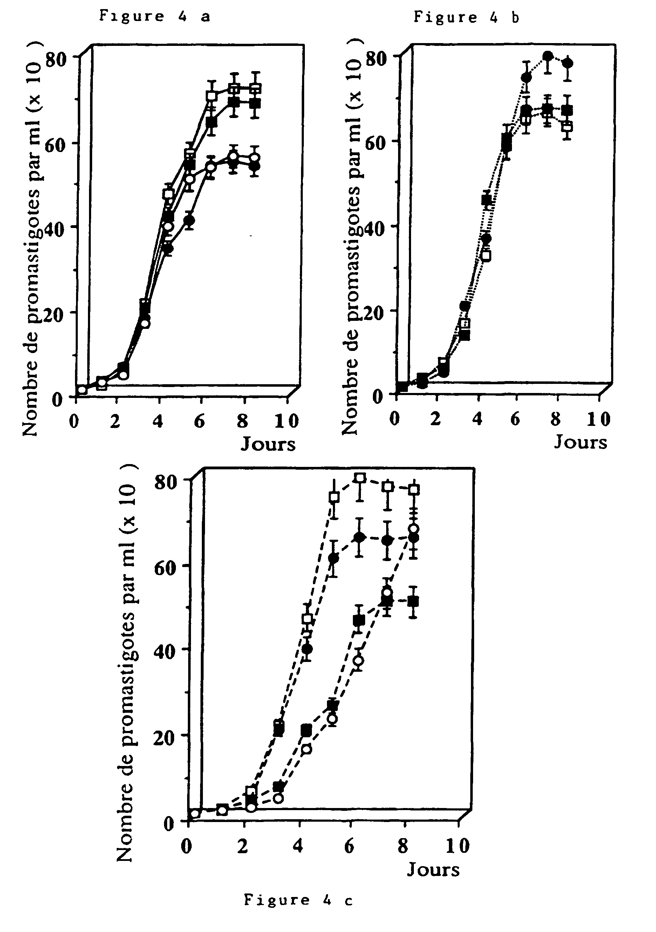

Les courbes de cinétique de culture des formes promastigotes d'espèces de leishmanies et de T. cruzi sont rapportées sur les figures 4a à 4c. Elles ont été établies après au moins 30 repiquages de 5 x 10⁶ parasites dans 10 ml de milieu M4.

La figure 4a donne les résultats obtenus avec L. braziliensis braziliensis (-□-), L. braziliensis guyanensis (-●-), L. braziliensis panamensis (-■-), et L. peruviana (-○-), la figure 4 b avec L. donovani infantum (--○--), L. donovani donovani (--●--) et L. donovani chagasi (--■--), la figure 4c avec L. mexicana mexicana (--□--), L. mexicana amazonensis (--●--), L. maior (--■--) et T. cruzi (--○--).

Les trois phases caractéristiques du cycle de croissance cellulaire observées classiquement au cours de la culture des formes promastigotes sont retrouvées : une phase de latence (2 jours), une phase exponentielle (6 jours) et une phase stationnaire (à partir du 7ème jour).

Les temps de doublement réel, calculés au cours de la phase exponentielle, sont sensiblement les mêmes pour les différentes cinétiques de culture. Ils varient de 23 à 30 heures selon les espèces de leishmanies alors qu'ils fluctuent de 16 à 21 heures pour les formes promastigotes correspondantes cultivées en présence de sérum de veau foetal dans les mêmes conditions d'inoculum, de volume de milieu d'aération.

Enfin, dans les conditions de ces expériences, les concentrations parasitaires déterminées à la confluence des cultures asériques (environ 7 jours) de formes promastigotes varient de 5,0 à 7,9 x 10⁷ par ml de milieu M4. Certaines espèces de leishmanies sont maintenues en routine par un repiquage hebdomadaire depuis plus de trois ans.

### c. Etude de l'infectivité in vitro et in vivo des formes promastigotes de culture asérique

Les formes promastigotes de culture asérique sont récoltées à 4°C par centrifugation à 2500 g. Le culot parasitaire est alors soumis à trois lavages, dans les mêmes conditions de centrifugation, en tampon PBS, pH 7,2. La concentration parasitaire est déterminée par comptage en cellule de Thoma.

### - Infectivité in vitro

On rapporte les résultats obtenus avec L. chagasi, L. donovani, L. amazonensis, L. mexicana et L. braziliensis

Selon le protocole décrit dans l'exemple 7, les macrophages péritonéaux de souris Balb/C sont infectés avec les formes promastigotes de phase stationnaire à raison de 10 parasites par macrophage.

L'analyse des cinétiques d'infectivité in vitro révèle qu'après 4 heures d'incubation, de 24 à 70 % des macrophages selon les espèces étudiées, présentent des formes promastigotes attachées à leur surface. Au cours de la cinétique, les pourcentages de macrophages infectés augmentent pour atteindre de 96 à 100 % à 48 heures selon les espèces de leishmanies.

Ces résultats démontrent que les formes promastigotes obtenues selon l'invention sont capables d'infecter les macrophages in vitro.

### Infectivité in vivo

On rapporte les résultats obtenus avec L. mexicana et L. amazonensis.

Des lots de 12 souris Balb/C sont respectivement infectées par voie sous-cutanée au niveau du coussinet plantaire de la patte arrière droite (la patte gauche servant de témoin) avec 5 10⁷ formes promastigotes (de phase stationnaire) selon l'invention de L. mexicana et de L. amazonensis. La taille de la lésion développée au point d'inoculation est déterminée au cours du temps.

Des lésions caractéristiques apparaissent dès la cinquième semaine d'infection. Leur taille atteint respectivement environ 5 et 6 mm à la quatorzième semaine pour L. mexicana et L. amazonensis

### Exemple 10 : Comparaison des formes amastigotes extracellulaires selon l'exemple 7 avec des formes amastigotes intracellulaires.

On rapporte dans cet exemple les résultats obtenus avec L. amazonensis, L. mexicana, L. brazilensis, L. chagasi et L. donovani.

### . obtention des formes extracellulaires

Les formes amastigotes de culture axénique des différentes espèces de leishmanies sont récoltées par centrifugation à 2500 g. Le culot parasitaire est alors soumis à trois lavages successifs par centrifugation (même condition) en tampon PBS, pH 7,2. Pour certaines études et notamment les tests d'agglutination par une lectine, une dissociation mécanique est nécessaire de façon à séparer les parasites naturellement agglutinés. La concentration parasitaire est déterminée par comptage en cellule de Thoma.

### . obtention des formes intracellulaires

Les formes amastigotes intracellulaires de L.amazonensis et de L. mexicana sont isolées de lésions développées au point d'inoculation chez des souris BALB/C ayant subi une injection sous-cutanée, au niveau du coussinet plantaire de la patte arrière droite, de 5 x 10⁷ formes promastigotes de phase stationnaire. Les lésions sont prélevées et broyées stérilement dans du tampon PBS, pH 7,2 contenant 2 % de glucose. Une première centri-fugation à faible vitesse (400 g) permet de se débarasser des tissus broyés. La suspension parasitaire est alors lavée trois fois en PBS par centrifugation à 2500 g. Les formes amastigotes intracellulaires sont comptées en cellule de Thoma.

Les amastigotes intracellulaires de L. chagasi et L. donovani sont obtenues à partir d'un broyat de rates provenant de hamsters infectés par 5 x 10⁷ formes promastigotes correspondantes, inoculées par voie intra-péritonéale. Ces formes sont isolées comme décrit ci-dessus.

### Etude morphologique

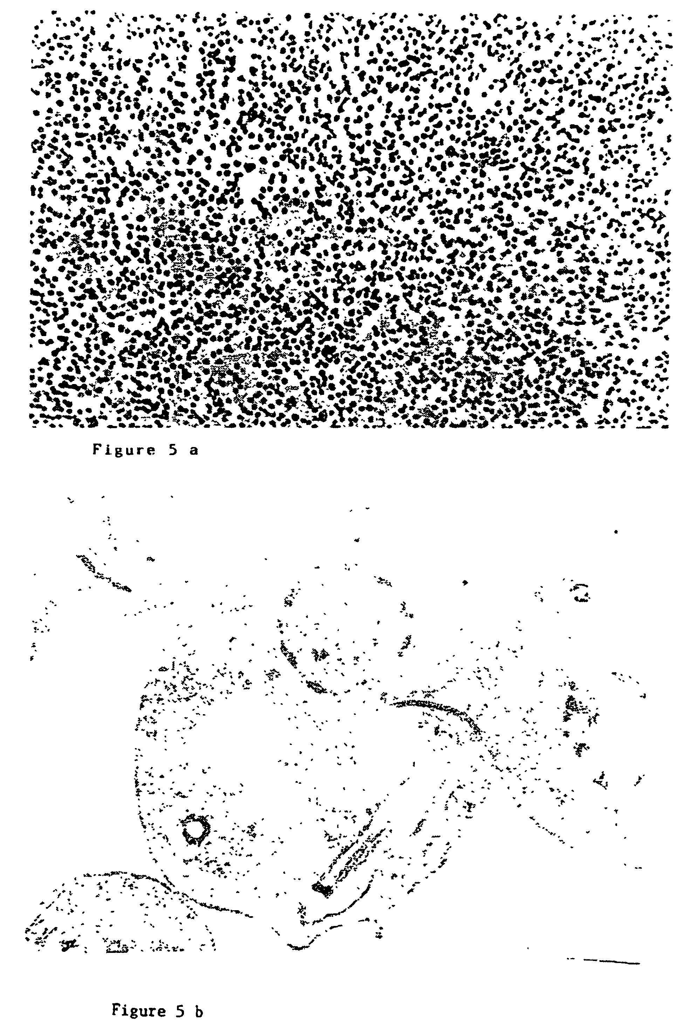

Les caractéristiques cytologiques des formes amastigotes de culture selon l'invention ont été comparées aux formes amastigotes intracellulaires en microscopie optique après fixation et coloration au Giemsa des parasites. A titre illustratif, on donne les résultats observés avec L. amazonensis sur la figure 5 a. On vérifie à l'examen de cette figure que les amastigotes de culture présentent des caractéristiques morphologiques générales des leishmanies : noyau et kinétoplaste bien individualisés, mais aussi des critères spécifiques des formes amastigotes : formes rondes ou ovoïdes de 2 à 5 µm sur le plus grand axe et absence du flagelle. La flèche portée sur la figure indique des formes amastigotes en voie de division.

Une étude ultrastructurale en microcospie électronique à balayage et à transmission a permis de confirmer l'analogie ultrastructurale entre les formes amastigotes intracellulaires et de culture (figure 5 b).

### Etude de l'infectivité in vitro des formes amastigotes de culture axénique selon l'invention

Des macrophages péritonéaux de souris BALB/C sont récupérés par lavage de la cavité péritonéale. Ils sont répartis dans des plaques de culture de 24 puits au fond desquels est déposée une lamelle de verre de 12 mm de diamètre, à raison de 2,5 x 10⁵ macrophages par puits dans 500 µl de RPMI 10 % SVF. Les plaques sont ensuite transférées à 36 ± 1°C, dans une atmosphère enrichie à 5% de CO₂, pendant environ 14 heures. Un lavage est effectué le lendemain pour éliminer les macrophages qui n'ont pas adhéré à la lamelle. Les puits sont ensuite infectés avec les formes amastigotes de phase exponentielle ou de phase stationnaire à raison de 5 parasites par macrophage.

Après 4 heures d'incubation à 36 ± 1°C, tous les puits sont lavés trois fois avec du milieu RPMI additionné de 10 % de SVF pour éliminer les parasites non attachés aux macrophages. Deux lamelles sont prélevées, lavées en PBS stérile puis séchées rapidement, fixées et colorées selon la technique de Giemsa, de façon à évaluer le pourcentage de macrophages infectés et pour certaines espèces de leishmanies, le nombre de parasites par macrophage. Cette opération est répétée après 24, 48 et 72 heures d'incubation. Les écart-types sont calculés sur deux valeurs correspondant chacune à trois lectures de 500 cellules.

L'étude des cinétiques d'infectivité in vitro montre qu'après 4 heures d'incubation, de 35 à 75,4 % de macrophages selon les espèces présentent des formes amastigotes attachées à leur surface. A partir de 24 heures, les pourcentages de macrophages infectés (contenant des formes amastigotes) augmentent et atteignent 100 % à 72 heures pour la plupart des leishmanies.

Ces résultats démontrent que les formes amastigotes obtenues selon l'invention sont capables de s'attacher aux macrophages, d'y pénétrer, de s'y multiplier et de coloniser d'autres macrophages.

L'étude comparative des cinétiques d'infection in vitro avec des formes amastigotes de leishmanies cutanées et de leishmanies viscérales intracellulaires correspondantes montre que les pourcentages de macrophages présentant des formes amastigotes attachées à leur surface (4 h) ainsi que les pourcentages de macrophages infectés au cours du temps (24, 48 et 72 h) sont très semblables.

### Etude de l'infectivité in vivo

Les protocoles d'infection expérimentale sont différents selon qu'il s'agit de leishmaniose cutanée ou de leishmaniose viscérale. On rapporte les résultats obtenus avec L. mexicana et L. amazonensis d'une part et L. chagasi et L. donovani d'autre part.

### Leishmaniose cutanée

5 x 10⁶ formes amastigotes intracellulaires et de culture (de phase exponentielle ou stationnaire selon l'invention) de L. mexicana et de L. amazonensis, contenues dans 25 µl de tampon PBS, sont respectivement injectées à douze souris BALB/C par voie sous-cutanée au niveau du coussinet plantaire de la patte arrière droite, la patte gauche servant de témoin. Les cinétiques d'infection sont suivies en mesurant, à l'aide d'un vernier de précision, la taille de la lésion développée au point d'inoculation.

Des lésions caractéristiques apparaissent au point d'inoculation entre la quatrième et la cinquième semaine d'infection. Leur taille augmente rapidement au cours du temps et atteint 6 à 7 mm à la neuvième semaine. Chez les souris infectées par L. amazonensis, des lésions ulcératives sont observées à partir de la septième semaine d'infection.

Une étude comparative des courbes d'infection expérimentale des formes amastigotes de culture selon l'invention et des formes intracellulaires montre une très bonne similitude.

### Leishmaniose viscérale

Des lots de douze hamsters dorés sont respectivement infectés par une injection intra-péritonéale (200 µl) de 5 x 10⁶ formes amastigotes intracellulaires et de culture (de phase exponentielle et stationnaire) selon l'invention de L. chagasi et de L. donovani. Tous les 21 jours, deux hamsters par lot sont sacrifiés. La rate ainsi qu'un lobe de foie sont prélevés et broyés stérilement dans 2 ml de tampon PBS. 500 µl de la suspension sont mis en culture dans deux types de milieu de culture (5 ml), le milieu RPMI additionné de 10 % de SVF à 26°C et le milieu M1 selon l'exemple 1 à 32°C. Des repiquages (dilution au tiers) sont réalisés tous les 4 jours. L'apparition de formes promastigotes et amastigotes est suivie dans le temps et la concentration parasitaire est estimée en microscopie photonique.

La mise en culture des broyats de rates correspondants au 21ème jour d'infection montre que, dès la deuxième subculture dans le milieu RPMI ci-dessus, deux à cinq formes promastigotes de L. chagasi par champs sont observées en microscopie optique. Avec L. donovani , on voit apparaître les premières formes promastigotes après quatre repiquages. Au 42ème jour de l'infection, de 2 à 5 formes promastigotes par champs sont visualisées dès le premier passage pour L. chagasi et lors du deuxième repiquage pour L. donovani. Au 63ème jour, plus de 10 parasites par champs sont dénombrés dès le premier repiquage pour les deux espèces de leishmanies. La mise en culture des broyats de rate des deux espèces de leishmaniose viscérale dans le milieu M1 32°C conduit à la survie, puis la multiplication, des formes amastigotes après 4 à 5 repiquages dans ce milieu.

Ces résultats démontrent, sans conteste, que les formes amastigotes de culture, des leishmanioses cutanées et viscérales, telles qu'obtenues selon l'invention, sont infectantes in vivo. Ces résultats sont confirmés en opérant en conditions asérique et en l'absence de macromolécules.

### . Modulation de l'infectivité in vitro et in vivo des formes amastigotes de culture en fonction de l'âge des parasites en culture.

De nombreux travaux mettent en évidence une modification de l'infectivité in vitro (vis-à-vis des macrophages péritonéaux) et/ou in vivo (dans un modèle expérimental) des formes promastigotes au cours de leur maturation en culture. Le développement séquentiel d'un stade peu infectant vers un stade très infectant a été démontré pour de nombreuses espèces de leishmanies.

On a donc comparé l'infectivité in vitro (vis-à-vis des macrophages péritonéaux de souris) et in vivo (dans un modèle expérimental) des formes amastigotes de culture axénique selon l'invention à différentes phases de leur croissance.

### Etude in vitro

On a comparé l'infectivité in vitro des formes amastigotes de phase exponentielle et de phase stationnaire de différentes leishmanies, telles qu'obtenues selon l'exemple 4.

Les formes amastigotes de phase exponentielle sont beaucoup moins infectantes que celles de phase stationnaire. Dès la 4ème heure d'incubation, les macrophages présentant des formes amastigotes de phase stationnaire attachées à leur surface sont environ deux fois plus nombreux. La détermination des pourcentages de macrophages infectés au cours des cinétiques d'infection révèle des différences du même ordre en faveur des formes amastigotes de phase stationnaire.

### Etude in vivo

De même, la comparaison des cinétiques d'infection in vivo des formes amastigotes de culture de phase exponentielle avec celles de phase stationnaire montre que les formes amastigotes de phase stationnaire sont plus virulentes que celles de phase exponentielle.

On démontre ainsi que l'augmentation de l'infectivité des formes amastigotes de culture de L. amazonensis, L. chagasi, et L. donovani, au cours de leur croissance, s'accompagne d'une perte d'agglutination par la lectine PNA (peanuts agglutinine).

### Exemple 11 : Extraits polypeptiques totaux des amastigotes de culture selon l'invention et comparaison avec ceux des amastigotes intracellulaires.

### . Dosage des protéines

Des culots de 10⁹ formes amastigotes de culture selon l'invention et intracellulaires (phase stationnaire), préalablement lavés, sont respectivement remis en suspension dans 500 µl de solution d'extraction (NaCl 0,1 % (P/V), 0,1 % de Triton X100 (V/V) et soumis à une série de sonication à froid de 3 pulses de 10 secondes espacés de 30 secondes. Le surnageant obtenu après une centrifugation à 14000 g constitue l'extrait polypeptidique total.

Cet extrait est soumis à un dosage des protéines selon la méthode de Bradford (BioRad Protein Assay Kit II).

### . Analyse électrophorétique

L'analyse électrophorétique en gel de polyacrylamide SDS-PAGE (conditions non réductrices) a été réalisée selon le protocole décrit par Laemmli U.K. et al, 1970, Nature, 227, 680. Les protéines sont concentrées dans un gel d'acrylamide à 5 %, puis séparées sur un gel d'électrophorèse à 10 %. 50 µg de protéines sont déposés par puits. Des protéines de poids moléculaires connus (Kit Low Weight, Biorad) servent de marqueurs. La migration s'effectue sous 85 volts pendant 2 heures à 4°C dans un tampon Tris/glycine, pH 8,3 contenant 0,1 % (P/V) de SDS.

La coloration au bleu de Coomassie permet de visualiser les profils polypeptidiques. Les polysaccharides libres ou complexés sont révélés par le réactif de Schiff.

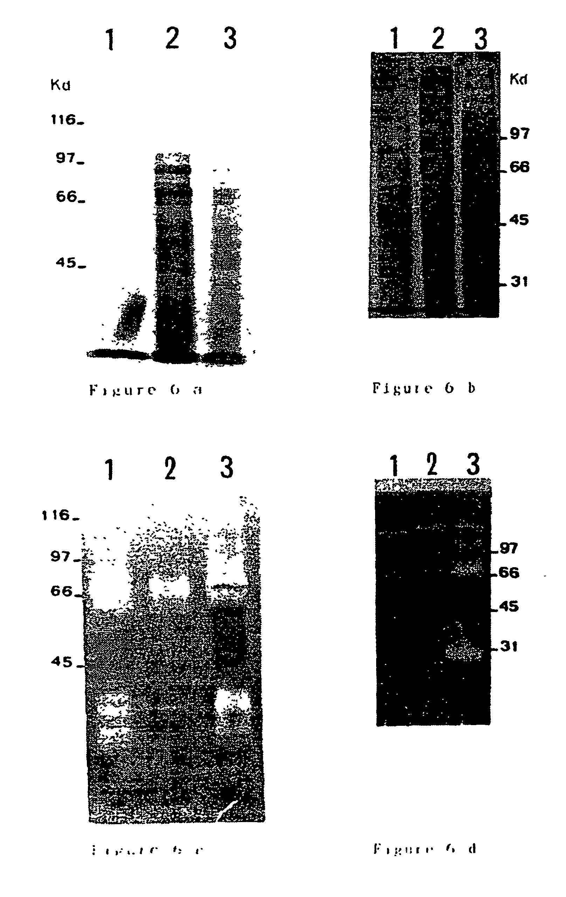

Des photos des gels obtenus sont représentées sur les figures 6a et 6b.

Dans la figure 6a, les profils 1, 2 et 3 sont respectivement les profils polypeptidiques totaux des formes promastigotes, des formes amastigotes de culture et des formes amastigotes de lésion, de L. mexicana.

Dans la figure 6b, les profils 1, 2 et 3 sont respectivement ceux des formes amastigotes de culture, des formes promastigotes et des formes amastigotes de lésion de L. amazonensis.

On observe une très bonne analogie entre les profils polypeptidiques totaux des formes amastigotes de culture axénique et ceux des formes amastigotes de lésion correspondantes (profils 3).

### Exemple 12 : Analyse des activités protéasiques en "gel d'empreinte".

5 mg de gélatine sont ajoutés à la solution d'acrylamide 10 % ci-dessus. Deux types de révélation sont alors effectuées : le gel est incubé 1 heure à température ambiante
- dans une solution de PBS 0,01 M, pH 7,2, contenant 2,5 % (V/V) de Triton X 100^{R} (pour éliminer le SDS) puis toute la nuit dans le tampon PBS seul :
- dans un tampon acétate, pH 5,5, contenant 2,5 % (V/V) de Triton X 100^{R}, puis environ 14 heures dans le tampon acétate contenant du dithiotréitol (DTT) (49 mM).

Les activités protéasiques sont révélées par une coloration du gel au bleu de Coomassie. Elles apparaissent alors en blanc sur fond bleu.

Les figures 6 c et 6 d représentent les photos des gels d'empreinte. Ces figures illustrent des activités protéasiques d'amastigotes de culture (profils 1), de promastigotes (profils 2) et d'amastigotes de lésion (profils 3), obtenues, respectivement, à partir des souches de L. mexicana et de L. amazonensis de l'exemple 7.

Comme pour les profils polypeptidiques totaux correspondants, on observe une très bonne analogie entre les profils d'activités peptidasiques des formes amastigotes de culture et des formes intracellulaires pour les deux espèces de leishmanies.

### Exemple 13 : Comparaison entre formes promastigotes et formes amastigotes de culture axénique selon l'invention.

### . Concentration protéique

La détermination des concentrations en protéines est réalisée par spectrophométrie à 595 nm selon la méthode de Bradford à partir d'un extrait polypeptidique total soluble correspondant à l'extraction d'un culot parasitaire de 10⁹ parasites.

Quelles que soient les espèces de leishmanies étudiées, les formes promastigotes contiennent environ deux fois plus de protéines que les formes amastigotes correspondantes (4,9 ± 0,7 mg pour 2,1 ± 0,3 mg par ml).

### . Comparaison des profils polypeptidiques totaux

### - de leishmanies cutanées.

On procède à une analyse électrophorétique en gel de polyacrylamide SDS-PAGE de ces extraits. Les photos des gels d'électrophorèse obtenus avec L. mexicana et L. amazonensis sont données sur les figures 6a et 6 b.

Comme le montre la figure 6 a, l'analyse comparative des profils polypeptidiques des formes promastigotes (profil 1) et des formes amastigotes de L. mexicana (profils 2 et 3) met en évidence d'importantes différences quantitatives et qualitatives.

On relèvera :
- une protéine prédominante d'un poids moléculaire apparent d'environ 65 kilo dalton (Kd en abrégé) spécifiquement révélée par les formes promastigotes ;
- trois polypeptides majeurs, ayant des poids moléculaires compris entre 55 et 90 Kd, révélés par les formes amastigotes, mais qui n'apparaissent pas présents dans l'extrait polypeptidique total des formes promastigotes.

Quelques différences sont aussi mises en évidence chez L. amazonensis :
- une bande correspondant à un poids moléculaire d'environ 58 Kd,
- plusieurs polypeptides situés dans des zones de poids moléculaires comprises entre 30 et 55 Kd, ainsi que
- des bandes présentant des poids moléculaires supérieurs à 90 Kd, qui apparaissent spécifiques des formes amastigotes (figure 6 b).

L'analyse des mêmes extraits parasitaires dans un gel de polyacrylamide coloré au réactif de Schiff démontre que la culture, selon l'invention, des formes amastigotes s'accompagne de l'expression croissante d'une molécule glycosylée de poids moléculaire d'environ 130 Kd.

Cette molécule apparaît peu ou pas représentée dans les profils correspondants de formes promastigotes.

Ces formes promastigotes révèlent en revanche la présence d'une molécule glycosylée avoisinant un poids moléculaire de 50 Kd.

### . Leishmanies viscérales

Les extraits polypeptidiques totaux de L. donovani et de L. chagasi sont soumis à une analyse électrophorétique en gel de polyacrylamide SDS-PAGE.

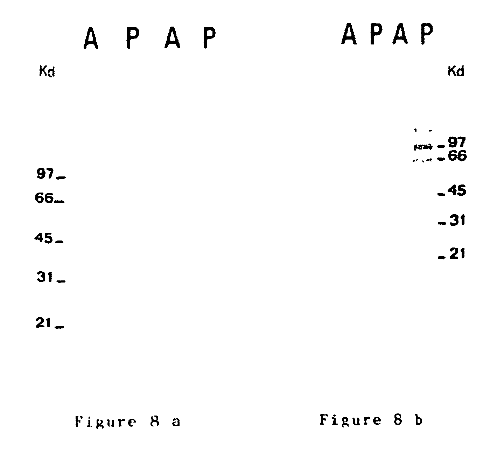

Les photos des profils polypeptidiques totaux respectivement obtenus sont donnés sur les figures 8A et 8B.

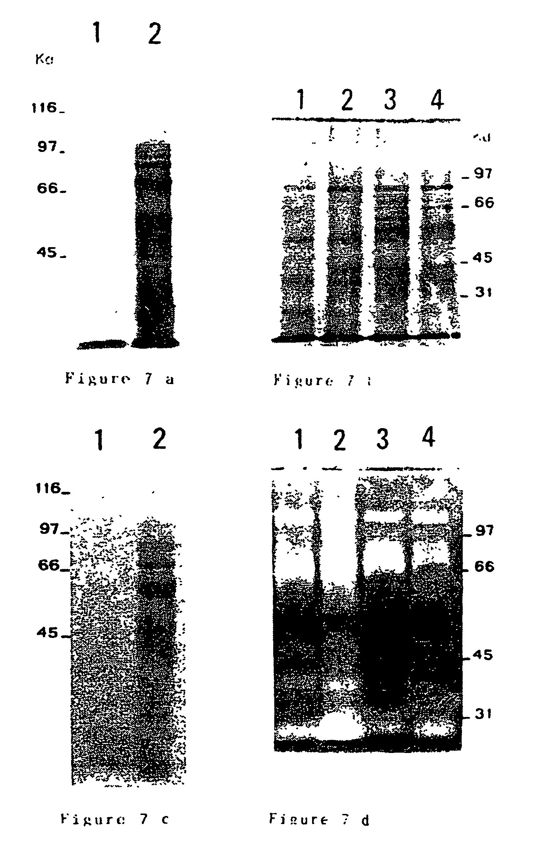

Sur la figure 7 a, les profils 1 et 2 correspondent respectivement aux formes promastigotes et aux formes amastigotes de culture de L. donovani et sur la figure 7 b, les profils 1 et 2 aux formes promastigotes et 3 et 4 aux formes amastigotes de culture L. chagasi.

On constate chez L. donovani (Figure 7 a), une protéine majeure révélée chez les formes amastigotes (profil 2) correspondant à un poids moléculaire d'environ 85 Kd et une bande d'environ 105 Kd, qui ne sont pas rencontrées chez les formes promastigotes (profil 1), qui révèlent comme pour L. mexicana une bande majeure spécifique d'environ 65 Kd.

Les formes amastigotes de phase exponentielle (profil 4) et stationnaire (profil 3) de L. chagasi (figure 7 b) présentent deux polypeptides majeurs correspondant à des poids moléculaires d'environ 55 et 60 Kd, qui sont absents des profils polypeptidiques totaux des formes promastigotes de phase exponentielle (profil 1) et de phase stationnaire (profil 2) correspondantes.

### . Comparaison des activités protéasiques

L'étude comparative des activités protéasiques des formes amastigotes et promastigotes correspondantes révèle d'une manière générale des profils d'activité qualitativement plus complexes et quantitativement plus importants chez les formes amastigotes de leishmanies, laissant apparaître des activités protéasiques spécifiques du stade amastigote.

### - de leishmanies cutanées

Comme le montre la Figure 6c, les formes promastigotes (profil 2) de L. mexicana expriment une activité majeure d'un poids moléculaire apparent d'environ 65 Kd, correspondant à la métalloprotéase de surface, alors que les formes amastigotes (profils 1 et 3) présentent :
- un complexe de bandes qui encadrent ce poids moléculaire (de 60 à 85 Kd),
- des activités de plus haut poids moléculaires (supérieurs à 110 Kd) avec des distances de migration variant selon le stade parasitaire,
   . trois activités protéasiques ayant des poids moléculaires compris entre 17 et 35 Kd révélées spécifiquement par les formes amastigotes.

Une bande mineure d'un poids moléculaire d'environ 25 Kd, devant correspondre à une activité cystéine protéase, mise en évidence seulement chez les formes promastigotes.

De telles différences sont aussi mises en évidence pour L. amazonensis (figure 6d). Les activités protéasiques majeures des formes promastigotes (profil 2), de poids moléculaires de 65 Kd et 115 Kd environ, présentent, chez les formes amastigotes (profils 1 et 3), des migrations électrophorétiques sensiblement plus rapides. Au moins trois activités protéasiques ayant des poids moléculaires compris entre 24 et 36 Kd sont révélées spécifiquement par les formes amastigotes ; seule une bande mineure d'un poids moléculaire d'environ 24 Kd et devant correspondre à une activité cystéine protéase, est mise en évidence chez les formes promastigotes de phase stationnaire.

### - de leishmanies viscérales

Chez L. donovani (figure 7 c), l'activité protéasique majeure des formes amastigotes de culture (profil 2) correspondant à un poids moléculaire d'environ 105 Kd n'est pas exprimée chez les formes promastigotes (profil 1). Ces dernières révèlent une bande mineure d'environ 65 Kd, qui est aussi faiblement rencontrée chez les formes amastigotes.

Pour L. chagasi (figure 7 d), deux activités protéasiques, de poids moléculaires de 28 et 110 Kd environ, sont présentes aussi bien chez les formes amastigotes de phases exponentielle (profil 1) et stationnaire (profil 2) que chez les formes promastigotes correspondantes (profils 3 et 4). Ces deux activités sont cependant plus intenses chez les formes amastigotes de phase stationnaire. Les formes amastigotes de phase exponentielle et stationnaire présentent des activités métalloprotéases plus complexes que celles exprimées par les formes promastigotes correspondantes. Enfin, deux activités de poids moléculaires de 35 et 48 Kd environ sont spécifiques des formes amastigotes.

### . Analyse antigénique

L'étude comparative des profils antigéniques des formes promastigotes et amastigotes a été réalisée par la technique d'immunoblotting à l'aide de sérums de lapins immunisés par des extraits polypeptidiques totaux de formes amastigotes de culture selon l'invention.

Les extraits polypeptidiques totaux des formes promastigotes et amastigotes sont séparés sur gel de polyacrylamide SDS-PAGE, transferrés sur une membrane de nitrocellulose qui est mise à incuber en présence des sérums d'immunisation correspondants. Les complexes antigène/anticorps sont révélés par un anticorps conjugué à la peroxydase dirigé contre les premiers anticorps.

La figure 8 a illustre les résultats obtenus par mise en contact des formes amastigotes de culture axénique et des formes promastigotes de L. amazonensis avec un immunsérum de lapin dirigé contre les formes amastigotes de L. amazonensis. On constate que quatre antigènes sont spécifiquement reconnus chez les formes amastigotes :
. un antigène majeur d'un poids moléculaire apparent de 62 Kd environ dont la migration est plus lente chez les formes promastigotes (65 Kd),
   - trois autres molécules correspondant à des poids moléculaires respectifs de 81, 58 et 35 Kd environ qui ne sont pas révélées chez les formes promastigotes,
   - deux antigènes de formes promastigotes ayant des poids moléculaires de 45 et 50 Kd environ, qui sont mieux reconnus par ce même immunsérum.

La figure 8 b, correspond aux amastigotes et promastigotes de L. mexicana vis-à-vis de l'immunsérum de lapin dirigé contre les formes amastigotes de L. mexicana. L'examen de cette figure montre que quatre antigènes correspondant à des poids moléculaires de 98, 90, 53 et 35 Kd environ sont révélés uniquement chez les formes amastigotes.

### . Comparaison de la réponse humorale vis-à-vis des formes amastigotes de culture axénique de l'invention et des formes promastigotes correspondantes.

L'intensité de la réponse en anticorps est évaluée par la technique d'immunofluorescence indirecte (IFI). Cette dernière consiste à détecter semi-quantitativement les anticorps circulants et présente la particularité d'utiliser comme substrat antigénique des parasites entiers fixés à la glutaraldéhyde 0,1 %.

### Etude avec des sérums d'immunisation

Les immunsérums de lapin anti-L.mexicana et anti-L. amazonensis sont testés en dilution sur les formes promastigotes et amastigotes de phase stationnaire de L. mexicana et de L. amazonensis.

Les sérums de lapin, prélevés avant immunisation ont servi à déterminer les seuils de positivité des tests. Ils sont de 1/2 pour L. amazonensis et 1/10ème pour L. mexicana.

Les résultats rapportés dans le tableau 9 suivant où l'antisérum de lapin anti-L. mexicana est appelé IS anti-Lma et celui de lapin anti-L.amazonensis est appelé IS anti-Lmm. Les lettres A et P représentent respectivement les formes amastigotes et promastigotes.

**Tableau 9**

| SERUMS / LAMES | Lma P | Lma A | Lmm P | Lmm A |
|---|---|---|---|---|
| IS anti-Lma A | - | + 1/5 | - | + 1/40 |
| IS anti-Lmm A | - | + 1/80 | - | + 1/40 |

L'examen de ce tableau montre que les deux immunsérums de lapin présentent uniquement des réactions positives avec les formes amastigotes de culture des deux espèces de leishmanies. La fluorescence est observée à la surface des formes amastigotes sous la forme d'un liseret et au niveau de la poche flagellaire. Ces résultats démontrent clairement que les formes amastigotes de leishmanies expriment à leur surface et au niveau de la poche flagellaire des antigènes spécifiques de stades communs aux deux espèces étudiées.

### Etude avec des sérums d'infection expérimentale

Des sérums de hamsters, infectés respectivement par des formes promastigotes de L. mexicana et de L. amazonensis sont prélevés à différents temps de l'infection expérimentale qui sont 30 (D30), 50 (D50) et 221 (D221) jours pour L. amazonensis et 30 (D30), 90 (D90) et 180 (D180) jours pour L. mexicana. Ces différents sérums sont testés en dilution sur les formes promastigotes et amastigotes de phase stationnaire de L. mexicana et de L. amazonensis. Les seuils de positivité sont déterminés à l'aide de sérums de hamsters sains. Il est de 1/20 quel que soit l'espèce et le stade parasitaire analysés.

Les résultats obtenus sont résumés sur le tableau 10 suivant où SIE anti-Lma et SIE anti-Lmm représentent respectivement les sérums d'infection expérimentale anti L. mexicana et anti-L. amazonensis.

**Tableau 10**

| SERA/LAMES | Lma P | Lma A | Lmm P | Lmm A |
|---|---|---|---|---|
| SIE anti-Lma D30 | - | 1/40 | - | - |
| SIE anti-Lma D50 | 1/20 | 1/160 | - | - |
| SIE anti-Lma D221 | 1/20 | 1/160 | - | 1/20 |
| SIE anti-Lmm D30 | - | - | - | 1/40 |
| SIE anti-Lmm D90 | - | 1/20 | 1/40 | 1/80 |
| SIE anti-Lmm D180 | - | 1/40 | 1/40 | 1/160 |

Les sérums d'infection précoce (D30) réagissent spécifiquement et uniquement avec les formes amastigotes de l'espèce homologue. D'une manière générale, les titres en anticorps obtenus vis-à-vis des formes amastigotes augmentent au cours des deux cinétiques d'infection. Les sérum D50 et D221 de L. amazonensis et D90 et D180 de L. mexicana révèlent aussi des réactions faiblement positives (1/20ème et 1/40ème respectivement) avec les formes promastigotes correspondantes. Les titres en anticorps obtenus sont toujours inférieurs à ceux révélés avec les formes amastigotes. Enfin les sérums D221 de L. amazonensis et D90, D180 de L. mexicana reconnaissent faiblement et uniquement les formes amastigotes de l'espèce hétérologue.

Ces résultats confirment l'existence d'antigènes spécifiques du stade amastigote. Ils démontrent aussi que la réponse humorale générée au cours des infections expérimentales est plus spécifiquement dirigée contre des épitopes exprimés par les formes amastigotes. Enfin, ils montrent que les antigènes de formes amastigotes sont plus aptes à détecter une infection précoce.

### Etude de la réponse humorale au cours de la leishmaniose canine

L'étude de la réponse humorale est réalisée en immunofluorescence indirecte sur 39 sérums de chiens leishmaniens. Elle consiste à comparer la réponse en anticorps vis-à-vis de deux substrats antigéniques, à savoir les formes promastigotes et amastigotes de phase stationnaire de L. chagasi. Deux groupes de sérums sont utilisés : 20 sérums présentant des titres anticorps inférieurs ou égaux à 1/160ème ("faiblement positifs") et correspondant vraisemblablement à des infections précoces et 19 sérums révélant des titres supérieurs ou égaux à 1/1280ème (fortement positifs), signant des infections bien établies.

Le seuil de positivité est déterminé à l'aide de 12 sérums de chiens indemnes de leishmaniose. Dans les conditions expérimentales utilisées de dilutions de conjugué fluorescent et de bleu Evans, quelque soit l'antigène figuré utilisé, le seuil de positivité est établi à 1/40ème.

Les résultats de cette étude sont représentés sur les tableaux 11 A et 11 B suivants où Ldc = L. chagasi, le tableau 9 A correspondant aux sérums dits faiblement positifs et le tableau 9 B aux sérums dits fortement positifs.

**Tableau 11 A**

| | | Différences exprimées en titres en anticorps obtenus sur les deux substrats antigéniques | | |
|---|---|---|---|---|
| Antigènes figurés comparés | Nombre de sérums | Ecart Moyen | Ecart minimum | Ecart maximum |
| Ldc A > Ldc P | 12 | 2,0 ± 1,2 | 1 | 4 |
| Ldc A = Ldc P | 3 | 0 | 0 | 0 |
| Ldc A < Ldc P | 5 | 2,2 ± 1,3 | 1 | 4 |

Parmi les 20 sérums "faiblement positifs", 12 d'entre eux présentent des titres en anticorps plus élevés, 3 des titres équivalents et 5 des titres inférieurs vis-à-vis des formes amastigotes de L. chagasi (tableau 11 A). Les écarts exprimés en nombres de titres en anticorps supérieurs ou inférieurs sont très importants et varient de 1 à 4 titres.

**Tableau 11 B**

| | | Différences exprimées en titres en anticorps obtenus sur les deux substrats antigéniques | | |
|---|---|---|---|---|
| Antigènes figurés comparés | Nombre de sérums | Ecart Moyen | Ecart minimum | Ecart maximum |
| Ldc A > Ldc P | 17 | 2,5 ± 1,6 | 1 | 6 |
| Ldc A = Ldc P | 2 | 0 | 0 | 0 |
| Ldc A < Ldc P | 0 | / | / | / |

Comme le montre le tableau 11 B, l'analyse comparative des sérums "fortement positifs" est encore plus convaincante. En effet, tous les sérums révèlent des réactions plus intenses avec les formes amastigotes de L. chagasi, avec des écarts du nombre de titres en anticorps supérieurs allant de 1 à 6.

Ce nouveau modèle expérimental permet de mettre en évidence une meilleure sensibilité de la technique d'immunofluorescence indirecte utilisant comme substrat antigénique les formes amastigotes de culture axénique en comparaison avec les formes promastigotes correspondates.

### Exemple 14 : Adaptation des formes amastigotes de culture en milieux complètements définis

Les formes amastigotes de diverses leishmanies ont été adaptées dans le milieu M3 selon l'exemple 3, parmi lesquelles celles de L. mexicana, L. amazonensis, L. braziliensis et L. chagasi.

Une adaptation progressive dans le milieu est réalisée comme suit : environ 10⁷ formes amastigotes de culture selon l'exemple 4 sont inoculées dans 5 ml de milieu contenant 25 % puis 50 % puis 75 % et enfin 100 % de milieu M4.

A chaque concentration, 4 à 5 repiquages sont effectués à raison de un, puis de deux passages par semaine.

Les cinétiques de culture des formes amastigotes de culture axénique et asérique des différentes espèces de leishmanies étudiées, après plus de 20 repiquages dans le milieu M3, sont analogues à celles déterminées dans le milieu M1.

### Exemple 15 : Réalisation du cycle parasitaire in vitro

### a. En condition axénique :

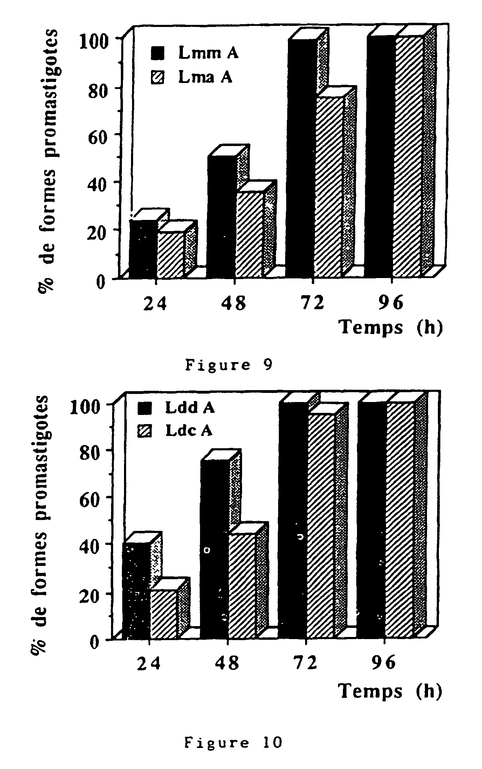

10⁷ formes amastigotes de phase exponentielle obtenues selon l'exemple 4 sont ensemencées dans 10 ml de milieu RPMI additionné de 10 % de SVF à 26°C. Aux différents jours de la culture, 20 µl de la suspension parasitaire sont déposés sur une lame, séchés rapidement puis fixés par du méthanol absolu pendant 2 minutes, colorés selon la méthode de Giemsa pendant 10 minutes. Le pourcentage de formes promastigotes est déterminé par comptage en microscopie optique (x 400) dans 20 champs successifs. Des repiquages successifs sont réalisés tous les 4 jours à raison de 10⁶ parasites par ml avant d'établir les courbes de cinétique de différenciation.
On a représenté sur les figures 9 et 10 le pourcentage de promastigotes obtenus, en fonction du temps (en heures) avec L. braziliensis 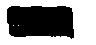 , L. guyanensis 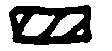 , L. panamensis 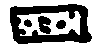 (figure 9) et L. chagasi 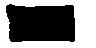 et L.donovani 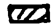 (figure 10).

L'examen de ces figures montre que la transformation des formes amastigotes de culture en formes promastigotes à 26°C est totale en 4 jours pour toutes les espèces de leishmanies étudiées.

Ces formes promastigotes, encore appelées promastigotes de primo culture ou de court terme, inoculées dans le milieu M1 à raison de 10⁶ parasites par ml, se différencient très rapidement (3 à 4 jours) en formes amastigotes à 32°C. La transformation est totale après 2 à 5 repiquages successifs selon les espèces de leishmanies étudiées.

Par ce procédé, il est donc possible d'obtenir in vitro les différents stades parasitaires des leishmanies et ceci beaucoup plus rapidement et facilement que par les techniques in vivo actuellement utilisées (infections expérimentales). De plus les formes parasitaires ainsi produites sont exemptes de tout contaminant cellulaire et sont capables de se multiplier in vitro (les formes amastigotes isolées de tissus infectés survivent 2 à 3 jours), ce qui permet de disposer d'une source abondante des deux stades parasitaires des principales espèces de leishmanies.

On a étudié les cinétiques d'infection expérimentale chez la souris Balb/C des formes promastigotes de phase exponentielle de court terme et de phase stationnaire de long terme et de court terme. Les résultats obtenus sur L. amazonensis ont montré qu'il est possible de restaurer l'infectivité des formes promastigotes de "long terme" en réalisant le cycle parasitaire in vitro. La même démonstration a été réalisée pour des leishmanies viscérales aussi bien in vitro que in vivo.

L'infectivité in vivo des formes amastigotes de culture a aussi été étudiée au cours des différents repiquages successifs (17, 59 et 143 repiquages). Les cinétiques d'infectivité obtenues ne sont pas significativement différentes en fonction du nombre de passages en culture. Ces résultats montrent que, contrairement aux formes promastigotes correspondantes, la culture à "long terme" des formes amastigotes de culture ne conduit pas à une perte de leur infectivité in vivo .

### b. En condition axénique et asérique

On rapporte les résultats obtenus avec L. chagasi et L. amazonensis. 10⁷ formes promastigotes de culture asérique de fin de phase exponentielle/début de phase stationnaire sont inoculées dans 10 ml de milieu M3 selon l'invention et incubées à 32 ± 1°C. Le pourcentage de formes amastigotes obtenu en fonction du temps est déterminé selon le protocole décrit précédemment. La différenciation des formes promastigotes en formes amastigotes est totale en 48 heures pour les deux espèces étudiées. Il est alors possible de cultiver les formes amastigotes en conditions axénique et asérique comme il est indiqué dans l'exemple 10.

10⁷ formes amastigotes de culture récemment transformées de formes promastigotes de culture asérique sont encemencées à 26 ± 1°C dans 10 ml de milieu M4 selon l'invention. La transformation des formes amastigotes en formes promastigotes est alors complète en 3 à 5 jours. Les formes promastigotes ainsi obtenues encore appelées promastigotes de primo-culture ou de court terme, sont maintenues en culture dans le milieu M4 par un repiquage hebdomadaire comme il est indiqué dans l'exemple 5, a.

Les procédés d'adaptation et de mise en culture, mis au point sur les principales espèces de leishmanies, ont été appliqués à la culture des formes amastigotes de souches de T. cruzi, agent de la maladie de Chagas.

On rapporte ci-après les résultats obtenus avec les souches SO 34 cl 1, Gamba cl 1 et SO 3 cl 1, typifiées isoenzymatiquement par l'analyse de plus de 15 loci. Ces souches ont été rapidement adaptées et cultivées dans le milieu M1 à 32°C à partir des formes trypomastigotes métacycliques (formes infectantes de l'insecte vecteur) correspondantes. Les formes trypomastigotes sont obtenues à partir des formes épimastigotes (formes de multiplication de l'insecte vecteur) cultivées en milieu LIT contenant 10 % de SVF, selon un protocole décrit par Contreras et al (1985, Mol. Biochem. Parasitol., 16, 315). 5 x 10⁷ formes épimastigotes sont incubées pendant 2 heures dans un milieu de synchronisation TAU (Tampon phosphate 8 mM, NaCl 190 mM, KCl 17 mM, CaCl₂ 2 mM, pH 6), puis dans un milieu de différenciation TAUP (TAU + L-proline 10 mM, Ac. L- aspartique 2 mM, L-glutamine 50 mM et D-glucose 10 mM) jusqu'à une totale transformation des formes épimastigotes en formes trypomastigotes. Environ 5 x 10⁷ formes trypomastigotes métacycliques sont ensemencées dans 10 ml de milieu M1 32°C. La différenciation en formes amastigotes est totale après 4 repiquages successifs avec les souches SO 34 et Gamba et 11 repiquages pour la souche S 03. Des souches de ce type sont cultivées en condition axénique de façon continue par un repiquage hebdomadaire et ont subi plus de 25 passages dans le milieu M1.

En microscopie optique, les amastigotes de culture de T. cruzi présentent des caractéristiques morphologiques générales des formes amastigotes intracellulaires. Une culture massive (200 ml) de la souche S0 34 cl 1) a été réalisée. Environ 10⁹ parasites lavés (trois lavages en tampon PBS pH 7,2) sont obtenus à partir de 50 ml de culture.

Différents essais de différenciation des formes amastigotes de culture des souches SO 34 et Gamba en formes épimastigotes (formes de multiplication de l'insecte vecteur) ou en formes trypomastigotes sanguicoles (formes infectantes de l'hôte) ont aussi été réalisés. Lorsque 5 x 10⁶ formes amastigotes de culture sont inoculées dans 10 ml de milieu M1 contenant 10 % de sérum de veau, puis incubées à 26°C ou à température ambiante pendant 48 heures, la culture contient plus de 90 % de formes épimastigotes. Par contre, quand une culture de formes amastigotes de fin de phase exponentielle est mise à incuber à 37°C en présence de 5% de CO₂ dans un milieu M1 contenant 30 % et plus de sérum de veau foetal pendant 4 jours, de 30 à 40 % des formes amastigotes sont transformées en formes trypomastigotes. Il est donc possible en jouant sur les conditions de température d'incubation et sur les concentrations en sérum de veau foetal de réaliser le cycle in vitro de Trypanosoma cruzi (formes épimastigotes, formes trypomastigotes métacycliques, formes amastigotes et formes trypomastigotes sanguicoles) en condition axéniques.

### Exemple 16 : Etude de l'effet leishmanicide direct sur les formes amastigotes de culture de l'oxyde nitrique, molécule effectrice de l'activation macrophagique.

On a décrit récemment des activités antimicrobiales de l'oxyde nitrique synthétisé par des macrophages activés selon une nouvelle voie métabolique conduisant à la synthèse par le macrophage activé de dérivés de l'oxyde nitrique (NO, NO₂⁻ et NO₃⁻) à partir de la L-arginine. Cette molécule effectrice de l'immunité non spécifique est aussi responsable d'activités antiparasitaires (effet trypanostatique chez les trypanosomes africains et sur Toxoplasma gondii et effet lytique sur Schistosoma mansoni et Leishmania major).

Grâce au nouveau modèle expérimental de l'invention, une activité leishmanicide directement dirigée contre les formes amastigotes de culture de différentes espèces de leishmanies a pu être démontré. De même, un des mécanismes conduisant à la lyse des formes amastigotes a été élucidé. En effet, les travaux effectués ont démontré que l'inhibition de la cis-aconitase (enzyme du cycle de Krebs), due à l'interaction du NO avec le groupement prosthétique Fe-S (Fer - soufre) de cette enzyme induit la lyse parasitaire. Ces résultats ont été obtenus en pulsant pendant 15 minutes du NO gazeux (1 %), véhiculé par de l'azote (99 %) dépourvu d'oxygène, directement sur les formes amastigotes de culture de L. mexicana, L. amazonensis, L. braziliensis et L. chagasi.

La cinétique de culture ainsi que la différenciation des formes amastigotes en formes promastigotes ont été suivies après ce traitement. Dans certaines expériences, des milieux contenant 100 µM de FeSO₄ (source de fer) ou de l'acide α-cétoglutarique (3 mM) et de la cis-aconitate (3 mM), additionnés immédiatement aux parasites traités par le NO restaurent la croissance des formes amastigotes.

L'étude comparative de différentes activités enzymatiques, et notamment celles du cycle de Krebs, sur les formes amastigotes traitées par le NO ou non a permis de mettre en évidence une inhibition de la cis-aconitase par le NO.

### Exemple 17 : Utilisation des exoantigènes de leishmanies dans une technique ELISA pour la détection des anticorps circulants dans la leishmaniose viscérale.

### a) Protocole

### Sensibilisation des plaques

Les surnageants de cultures axéniques et asériques de formes promastigotes de L.infantum (6, 15 et 21 jours) sont collectés par centrifugation et filtrés sur membrane Millipore^{R} 0,22 µm. Ils sont dilués au demi en tampon carbonate (0,5 M, pH 9,6) de façon à obtenir des concentrations respectives de 3,5, 4,6 et 7,0 µg/ml (équivalent protéines).

100 µl de ces solutions sont déposés dans chaque alvéole des plaques de microtitration. La sensibilisation dure 2 heures à 37°C, puis une nuit à 4°C.

### Lavages

On procède à 3 lavages de 5 minutes par retournement sur papier filtre en tampon de lavage (PBS 0,01 M, pH 7,2 contenant 0,05 % Tween 80).

### Saturation des sites de fixation non-spécifiques

100 µl de tampon bloquant (tampon de lavage contenant 0,25 % de gélatine) sont répartis dans chaque alvéole, puis on soumet le mélange à incubation 30 minutes à 37°C.

### Lavages

On procède à 3 lavages de 5 minutes en tampon de lavage.

### Distribution des sérums

Les sérums de chien ou humains sont dilués au 1/200 en tampon bloquant. 100 µl de sérum sont déposés par cupule. L'incubation est réalisée durant 30 minutes à 37°C.

### Lavages

On procède à 3 lavages de 5 minutes en tampon de lavage.

### - Dépôt de l'anti-immunoglobine correspondante marquée à la peroxydase.

Ce réactif, dilué au 1/500 est déposé à raison de 100 µl par alvéole. Le mélange est soumis à incubation 30 minutes à 37°C.

### Lavages

On procède à 5 lavages de 5 minutes en tampon de lavage.

### Révélation de l'activité peroxydasique

200 µl de réactif (1 mg ABTS dans 12 ml de tampon citrate 50 mM, pH 4 additionné de 5 µl d'eau oxygénée, 30 vol.) sont délivrés par cupule et les préparations soumises à incubation 30 minutes à température ambiante.

### Lecture

On utilise un spectrophotomètre à 414 nm (coloration verte).

### b) Résultats

Les différences obtenues entre les moyennes des densités optiques des sérums négatifs et positifs (en immunofluorescence indirecte) permettent de mettre en évidence les infections à L. infantum (voir tableau 12).

**Tableau 12**

| | Sérums négatifs | Sérums positifs |
|---|---|---|
| Surnageant 6 jours | 0,128 + 0,025 | 0,313 + 0,035 |
| surnageant 15 jours | 0,080 + 0,012 | 0,293 + 0,020 |
| surnageant 21 jours | 0,062 + 0,010 | 0,257 = 0,039 |

### Exemple 18 : Production des antigènes d'excrétion/sécrétion à partir des surnageants de culture métabolisés par les différents stades parasitaires des leishmanies.

Les surnageants métabolisés par les parasites de culture asérique, obtenus à différents temps de la culture, sont séparés des formes promastigotes ou amastigotes par centrifugation puis filtration sur membrane Millipore de 0,22 µm traitée préalablement à la bacitracine (1 mg/ml). L'échantillon est alors concentré par ultrafiltration sur Mini Ultrassette (Filtron, seuil de coupure : 3 kDa), dialysé une nuit contre de l'eau distillée et enfin lyophilisé. Ce procédé permet de concentrer plus de 400 fois les surnageants de culture.

Les dosages protéiques sont effectués par spectrophométrie à 595 nm selon la méthode de Bradford (Biorad Protein Assay Kit II). Selon les espèces de leishmanies, de 1 à 4 µg équivalent protéine d'antigène sont ainsi obtenus par ml de surnageant non concentré.

### Caractérisations biochimiques et immunologiques des antigènes d'excrétion/sécrétion de L. infantum et de L. amazonensis :

Les exoantigènes parasitaires sont des molécules solubles libérées naturellement dans le sang des hôtes infectés et dans les surnageants de cultures in vitro de ces parasites. Il faut faire la distinction entre les vrais exoantigènes (AES) résultant du métabolisme des parasites, et les antigènes résultant de leur lyse naturelle ou artificielle.

Selon l'invention, une incorporation de méthionine ³⁵S (1,85 MBq) est réalisée à partir du troisième jour (début de la phase exponentielle) de la culture de formes promastigotes de L. amazonensis (MHOM/FE/16/JAP-78 : leishmaniose cutanéo-diffuse) et de L. infantum (MOHM/MA/67/IT-263 clone 2 : leishmaniose viscérale humaine) inoculées à raison de 5 x 10⁵ parasites/ml dans un milieu synthétique exempt de macromolécule. Les AES et les extraits polypeptidiques parasitaires totaux (EPT) sont analysés en SDS-PAGE (polyacrylamide 10 %), en conditions non réductrices.

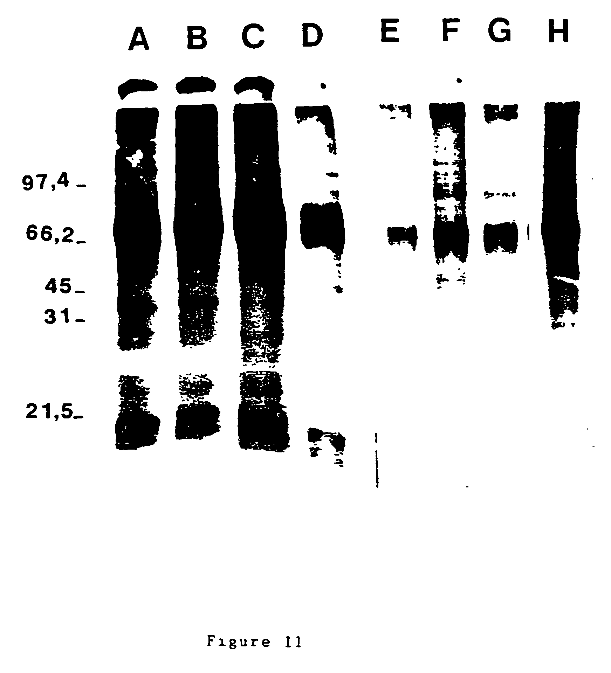

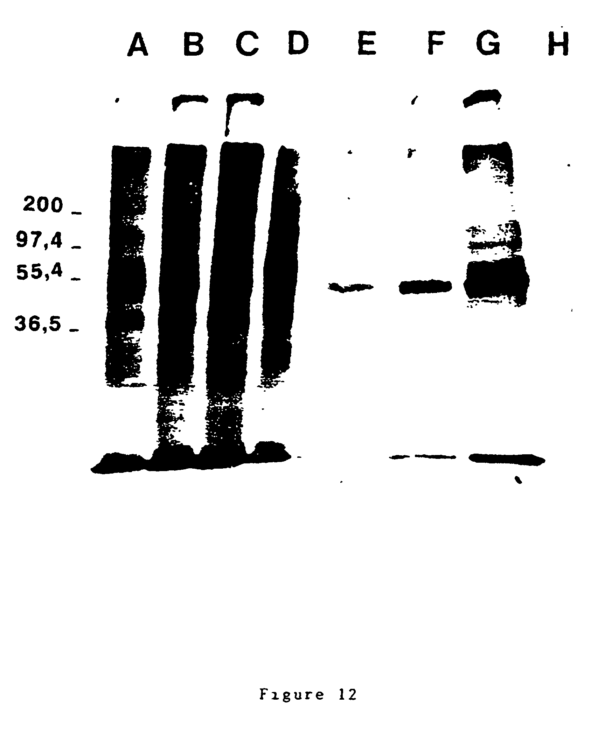

Les cinétiques d'incorporation de [³⁵S]-méthionine par les protéines totales ou d'excrétion/sécrétion de L. infantum et L. amazonensis sont respectivement rapportées sur les figures 11 et 12.

Sur la figure 11, A à D correspondent aux protéines totales extraites 24 h (A), 48 h (B), 72 h (C) et 96 h (D) après l'addition de [³⁵S]- méthionine ; E à H aux protéines excrétées/sécrétées prélevées 24 h (E), 48 h (F), 72h (G) et 96h (H) après l'addition de [³⁵S]-méthionine.

Sur la figure 12, A à D correspondent aux protéines totales extraites 24 h (A), 48 h (B), 72 h (C) et 96 h (D) après l'addition de [³⁵S] méthionine ; E à H aux protéines excrétées/sécrétées prélevées 24 h (E), 48 h (F), 72 h (G) et 96 h (H) après l'addition de [³⁵S] méthionine.

Les profils radiomarqués des AES sont beaucoup moins complexes que ceux des EPT quelque soit l'espèce étudiée. Seules 16 protéines d'excrétion-sécrétion sont révélées aussi bien pour L. infantum que pour L. amazonensis.

Des immunoprécipitations (IP) ont été réalisées :
- avec des sérums d'infections naturelles humaines ou canines vis à vis des AES et des EPT (6jours de culture) de L. infantum
- avec des sérums d'infestation expérimentale chez le hamster vis à vis des AES et EPT (6 jours de culture) de L. amazonensis
- avec des sérums d'immunisation chez le lapin vis-à-vis des AES et EP (6 jours de culture) de L. amazonensis.

Les résultats démontrent une immunogénicité des AES au cours d'infections naturelle ou expérimentale. Pour les deux parasites, certains antigènes sont reconnus uniquement au niveau des EPT alors que d'autres sont propres aux AES. Pour L. infantum, une similitude de réponse humorale est observée pour 5 des 6 sérums humains étudiés (2 doublets à environ 70 kDa et environ 45 kDa) vis-à-vis des AES et des EP ; le sérum de chien reconnaît des antigènes différents (antigènes majeurs à environ 90 et 66 kDa). Chez le hamster, il existe une évolution de la réponse humorale vis à vis des AES de L. amazonensis au cours de l'infection expérimentale. Les antigènes reconnus lors d'une infection expérimentale ou naturelle sont différents de ceux qui sont immunoprécipités par un sérum d'immunisation anti-EPT chez le lapin.

Ces résultats montrent que les AES sont capables d'induire une réponse anticorps au cours de l'infection par L. amazonensis et L. infantum. Compte tenu de leur immunogénicité, les AES constituent des outils de grand intérêt pour les leishmanioses dans les domaines du diagnostic et de la vaccination.

### Exemple 19 : Production d'anticorps monoclonaux contre les antigènes d'excrétion/sécrétion de L.amazonensis

### Protocole utilisé

### Immunisation

2 µg d'antigène (surnageant de culture asérique métabolisé par des formes promastigotes de fin de phase exponentielle de L. amazonensis, concentré 200 fois et dialysé) sont injectés par voie sous-cutanée au niveau du coussinet plantaire de la patte arrière. La préparation d'antigène injecté est appelée ci-après "Ag".

Un protocole d'immunisation rapide chez la souris BALB/e est réalisé comme suit : J₋₁₀ : Ag + Adjuvant complet de Freund (ACF) (V/V) ; J₋₇ et J₋₄ : Ag + AIF ; J₀ ; récupération des cellules des ganglions poplytés drainant la patte arrière.

### Fusion

Les cellules myélomateuses (X 63) sont mises à fusionner (J₀) avec les cellules lymphocytaires dans un rapport 1/2 en présence de poly-éthylène glycol 4000 (PEG 50%).

### Criblage

A J₊₇, 442 puits (38 %) ont présenté au moins un clone cellulaire en développement et 66 (5,7 %) des cellules à confluence. Les surnageants de cellules à confluence sont testés soit en immunofluorescence Indirecte (IFI), soit en test ELISA. 19 d'entre eux se sont révélés positifs en IFI, 9 en ELISA et 7 dans les deux tests. Au total, plus de 1000 puits ont été screenés. En boîtes de 96 puits, en boîtes de 24 puits, et en boites de 25 cm2, 120, 47 et 36 hybridomes se sont révélés respectivement sécréteurs (révélé par l'IFI)

### Clonage

Les hybridomes sécréteurs caractéristiques ont été clonés par dilution limite, 4 clones ont fait l'objet d'une étude particulière (B3, C7, D9 et F5).

Les résultats obtenus sont donnés dans le tableau 13.

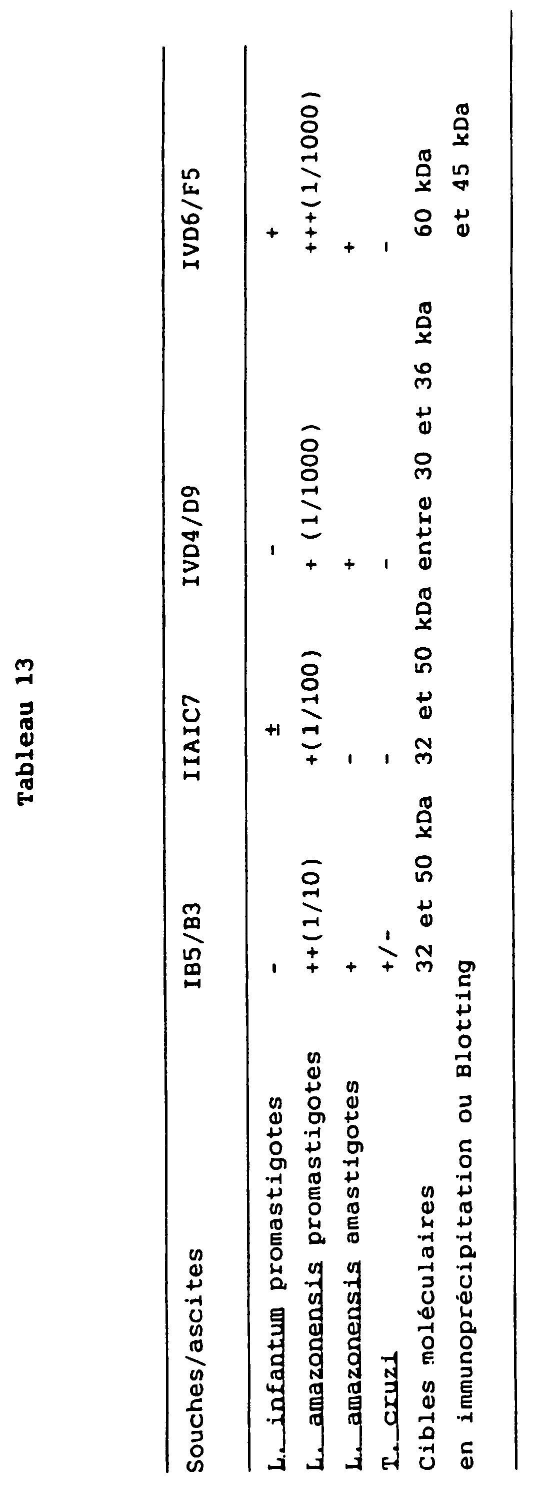

### Exemple 20 : Obtention de formes amastigotes résistantes à un médicament

Des formes promastigotes de L. mexicana résistantes à la pentamidine sont obtenues par pression médicamenteuse in vitro et présentent un indice de résistance d'environ 30. Elles ont ensuite été transformées selon le procédé de culture déjà décrit en formes amastigotes de culture correspondantes qui, après détermination de l'IC 50 conservent la résistance à la pentamidine induite chez les formes promastigotes.

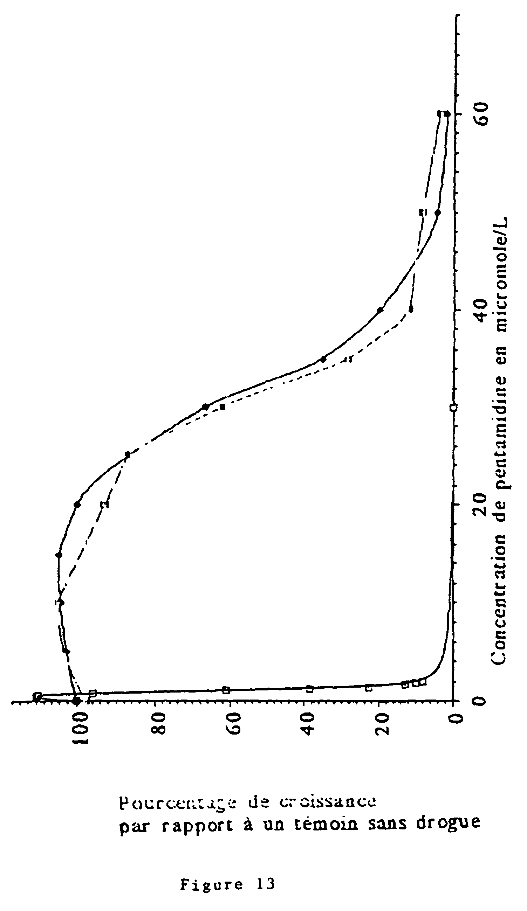

La figure 13 donne la variation du pourcentage de croissance par rapport à un témoin sans drogue en fonction de la concentration de pentamidine en µmole/l (la courbe 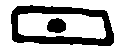 correspond aux promastigotes sauvages de L. mexicana, la courbe ◆ aux formes promastigotes résistantes, et la courbe 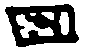 aux formes amastigotes résistantes. (Ic 50 : concentration de pentamidine pour laquelle on observe une inhibition de croissance de 50 % : Ic 50 sauvage 1,2 ± 0,05 µM, Ic 50 amastigote résistant 32 ± 1µM et Ic 50 promastigote résistant 33 ± 1 µM. L'indice de résistance est égal à Ic50 de la souche résistante /Ic50 de la souche sauvage. Pour les promastigotes la valeur est de 27 et pour les amastigotes de 26.

Grâce à la réalisation du cycle évolutif in vitro, l'invention fournit des formes amastigotes de leishmanies résistantes à un médicament. Ce nouveau modèle expérimental permet d'étudier très précisément le ou les mécanisme(s) impliqué(s) dans l'induction de la chimiorésistance chez le stade parasitaire présent chez l'hôte infecté.

## Revendications

1. Procédé pour la culture in vitro de différents stades de parasites tissulaires choisis parmi les leishmanies et T.cruzi, **caractérisé en ce qu'**il est réalisé en continu, dans un milieu de culture totalement défini, monophasique, liquide, axénique et asérique, dépourvu de macromolécules, c'est-à-dire de molécules non dialysables à un seuil de coupure de 3 kDa, ce milieu de culture comprenant un milieu pour la culture des cellules d'insectes, additionné de sels inorganiques de type sels de Hanks, d'acides aminés comme la L-glutamine, et de sucres, tamponné, pour l'obtention de formes amastigotes, à un pH de 5,5 à 6,5 avec une osmolarité d'au moins 400 milli osmoles/kg de liquide, et en particulier de 400 à 550 milli osmoles/kg de liquide, ou à un pH de 7 à 7,5 pour l'obtention de formes promastigotes, avec une osmolarité d'au moins 300 milli osmoles/kg de liquide, et en particulier de 300 à 380 milli osmoles/kg de liquide.

2. Procédé selon la revendication 1, **caractérisé en ce que** pour la culture des stades amastigotes, on utilise un milieu 199H M.

3. Procédé selon la revendication 1 ou 2, **caractérisé en ce qu'**au moment de l'utilisation, on ajoute au milieu de base des agents anti-oxydants comme l'hémine, des agents réducteurs, comme le glutathion réduit, la L-cystéine, ainsi que des vitamines ou l'acide bathocuproïne sulfonique.

4. Procédé selon l'une quelconque des revendications 1 à 3, **caractérisé en ce que**, par rapport au milieu final, le milieu de culture cellulaire représente environ 8 à 15 % (V/V), les acides aminés sont présents à raison de 4 à 8 % (P/V), les sucres, à raison d'environ 2 à 4 % (P/V), les agents anti-oxydants, comme l'hémine, sont présents à raison de 0,0002 à 0,0015 % (P/V), le glutathion à raison de 0,01 à 0,05 % (P/V) et la L-cystéine à raison de 0,25 à 0,50 % (P/V), l'acide bathocuproïne sulfonique, à raison d'environ 0,004 à 0,008 % (V/V), les vitamines représentant 1 % à 5 %.

5. Procédé selon la revendication 4, **caractérisé en ce que** le milieu de culture représente 10 % (V/V), les acides aminés 5 à 6 % (P/V), les sucres 2 à 3 % (P/V), les agents anti-oxydants, comme l'hémine, 0,0005 % (P/V), le glutathion 0,025 %, la L-cystéine 3 % (P/V), l'acide bathocuproïne sulfonique 0,005 % (V/V), les vitamines 2 % (V/V).

6. Procédé selon la revendication 1, **caractérisé en ce que** pour la culture des stades promastigotes, on utilise un milieu de culture élaboré à partir d'un milieu de culture de cellules, tel que le milieu RPMI 1640, auquel on ajoute des acides aminés tels que la L- glutamine, ce milieu étant tamponné à une valeur de 7 à 7,5 et étant également additionné d'un autre milieu de culture approprié pour la culture de cellules d'insectes, spécialement le milieu 199H M, renfermant des sels inorganiques tels que les sels de Hanks, et des agents anti-oxydants, tels que l'hémine.

7. Procédé selon la revendication 6, **caractérisé en ce que** le milieu 199H M est utilisé à raison d'environ au moins 2 % (V/V), et l'hémine bovine de 0,0001 à 0,0015 % (P/V).

8. Procédé selon la revendication 7, **caractérisé en ce que** le milieu 199H M est utilisé à raison de 2 % à 10 % (V/V) et l'hémine bovine de 0,0005 % (P/V).

9. Procédé selon l'une quelconque des revendications 1 à 8, **caractérisé en ce qu'**on ensemence des formes promastigotes de fin de phase exponentielle dans ledit milieu de culture, à raison de 10⁶ à 10⁷ formes promastigotes/ml de milieu, on réalise l'adaptation et la culture à une température de l'ordre de 28 à 36°C, et notamment autour de 32° C, à un pH de 5,5 à 6,5, de manière à obtenir une transformation complète des promastigotes en amastigotes

10. Procédé selon la revendication 9, pour la production massive et continue in vitro de formes promastigotes, de parasites **caractérisé par** l'ensemencement des formes amastigotes obtenues, à raison de 10⁶ à 10⁷ amastigotes/ml de milieu, leur culture à un pH de 7 à 7,5 dans un milieu de culture classique pour le développement de formes promastigotes en amastigotes.

11. Procédé selon la revendication 10, **caractérisé en ce qu'**on utilise des formes amastigotes de fin de phase exponentielle.

12. Procédé selon la revendication 10 ou 11, **caractérisé en ce que** les formes promastigotes obtenues sont utilisées pour l'étape d'ensemencement dans un milieu de culture pour produire des amastigotes selon la revendication 9.

13. Procédé selon l'une des revendications précédentes, **caractérisé en ce que** les amastigotes sont de T.cruzi et qu'on met en oeuvre des formes trypomastigotes à la place des formes promastigotes.

14. Les polypeptides d'excrétion/sécrétion directement obtenus,par concentration et dialyse, à partir des surnageants des milieux de cultures d'amastigotes ou de promastigotes de leishmanies ou de T.cruzi réalisées selon le procédé de l'une quelconque des revendications 1 à 13.

15. Polypeptides d'excrétion/sécrétion, selon la revendication 14 **caractérisés par** des PM dans des zones de 40 à 60 kDa et 65 à 90 kDa, telles qu'identifiées par marquage méthionine et analyse des extraits marqués en SDS-PAGE, respectivement pour *L.infantum* et *L.amazonensis*.

16. Anticorps polyclonaux dirigés contre des polypeptides antigéniques d'excrétion/sécrétion de la revendication 14 ou 15, directement obtenus à partir des surnageants des milieux de cultures d'amastigotes ou de promastigotes de leishmanies ou de T.cruzi, réalisées selon le procédé de l'une quelconque des revendications 1 à 13, ayant des PM de 32 à 50 kDa, 30 à 36 kDa, 45 à 60 kDa.

17. Anticorps monoclonaux tels qu'obtenus par fusion de cellules myélomateuses avec les lymphocytes, en particulier de la rate ou des ganglions d'un animal préalablement immunisé par injection de polypeptides selon la revendication 14 ou 15, criblage des surnageants des hybridomes obtenus, par exemple selon la technique E.L.I.S.A. ou I.F.I., de manière à révéler les anticorps dirigés spécifiquement contre une forme parasitaire d'une espèce, et souches d'hybridomes sécrétant ces anticorps monoclonaux.

18. Anticorps monoclonaux générés chez la souris contre les peptides d'excrétion/sécrétion d'amastigotes de *L.infantum* , de *L.amazonensis* ou de *T.cruzi* selon la revendication 15.

19. Utilisation comme modèle expérimental des formes parasitaires des leishmanies et de T.cruzi présentes dans l'hôte infecté, en particulier pour le criblage in vitro de médicaments et pour tester la sensibilité aux médicaments, le procédé de criblage comportant avantageusement :
- la mise en contact avec les produits à tester des formes parasitaires, telles que cultivées selon le procédé de l'une des revendications 1 à 13.
- l'incorporation de nucléotides ou d'acides aminés marqués par un groupe radioactif, par exemple de la thymidine tritiée, pour déterminer l'activité des produits à tester ou la réalisation de tests de viabilité.

20. Kit pour le criblage de produits susceptibles d'être utilisés pour le traitement de parasitoses, plus spécialement de leishmanies ou de la maladie de Chagas, **caractérisé en ce qu'**il comprend :
- un support tel qu'une plaque multipuits renfermant les formes parasitaires telles qu'obtenues par le procédé selon l'une quelconque des revendications 1 à 13,
- les réactifs pour déterminer l'activité médicamenteuse des produits testés sur les formes parasitaires.

21. Méthode de détection in vitro d'une parasitose, en particulier d'une leishmaniose ou de la maladie de Chagas, chez l'homme ou l'animal, ou de leur détection et identification chez l'insecte vecteur, par détection des anticorps circulants, **caractérisée en ce qu'**elle comprend :
- la mise en contact d'un échantillon biologique provenant de l'homme ou de l'animal à examiner avec une forme parasitaire telle qu'obtenue par le procédé selon l'une quelconque des revendications 1 à 13, un polypeptide d'excrétion/sécrétion selon la revendication 14 ou 15,
- la détection du complexe immunologique éventuellement formé.

22. Kit pour la détection in vitro de parasitoses selon la revendication 21, **caractérisé en ce qu'**il comprend :
- les réactifs antigéniques sous forme immobilisée, à savoir les formes parasitaires telles qu'obtenues selon l'une quelconque des revendications 1 à 13, les polypeptides d'excrétion/sécrétion selon la revendication 14 ou 15,
- un témoin positif, constitué par un sérum de titre connu,
- un témoin négatif, ainsi que
- les tampons et réactifs utilisables pour la révélation de la réaction immunologique.

23. Méthode de détection in vitro d'une parasitose chez l'homme ou l'animal, en particulier d'une infection par des leishmanies ou T.cruzi, par détection des antigènes circulants, **caractérisée en ce qu'**elle comprend :
- la mise en contact d'un échantillon biologique provenant de l'homme ou de l'animal à examiner avec des anticorps selon l'une des revendications 16 à 18, ou des fragments de ces anticorps, immobilisés sur un support non immunogène, dans des conditions appropriées pour la production d'un complexe antigène-anticorps,
- la mise en évidence de la formation du complexe.

24. Kit pour la détection in vitro d'une parasitose selon la revendication 23, **caractérisé en ce qu'**il comprend
- une phase solide appropriée servant de support pour la réaction immunologique, telle qu'une plaque de micro-titration,
- une préparation d'anticorps selon l'une des revendications 16 à 18, ou de fragments de ces anticorps immobilisés sur un support, avec, le cas échéant,
- un témoin positif, constitué par un sérum de titre connu,
- un témoin négatif, ainsi que
- les tampons et réactifs utilisables pour la réalisation de la réaction immunologique, et notamment l'anticorps homologue marqué.

25. Compositions de vaccins contre des parasitoses, en particulier des infections par des leishmanies ou T.cruzi, **caractérisées en ce qu'**elles sont élaborées à partir des polypeptides d'excrétion/sécrétion selon la revendication 14 ou 15, des formes promastigotes telles qu'obtenues selon le procédé de l'une quelconque des revendications 1, 8 à 10, des formes amastigotes telles qu'obtenues selon le procédé de l'une quelconque des revendications 1 à 9 et 11 ou 12, en association avec un véhicule et/ou un adjuvant d'administration.

26. Compositions de vaccins selon la revendication 25, **caractérisées en ce qu'**elles sont élaborées à partir d'amastigotes de L. infantum ou de L. chagasi.

27. Compositions de vaccins selon la revendication 25 ou 26, à des fins immunoprophylactiques ou immunothérapeutiques.

## Patentansprüche

1. Verfahren zur in vitro-Kultur von verschiedenen Stadien von Gewebeparasiten ausgewählte aus den Leishmanien und T.cruzi, **dadurch gekennzeichnet, daß** es kontinuierlich in einem vollständig definierten einphasigen flüssigen axenischen und aserischen Kulturmedium durchgeführt wird, das frei von Makromolekülen, das heißt bei einer Schnittschwelle von 3 kDa nicht dialysierbaren Molekülen durchgeführt wird, wobei dieses Kulturmedium ein Medium für die Kultur von Insektenzellen umfaßt, mit Zusatz von anorganischen Salzen vom Typ Hanks-Salze, Aminosäuren wie L-Glutamin, und Zucker umfaßt, das zur Gewinnung von Amastigoten-Formen auf einen pH von 5,5 bis 6,5 mit einer Osmolarität von mindestens 400 Milliosmolen/kg Flüssigkeit und besonders 400 bis 550 Milliosmolen/kg Flüssigkeit oder zur Gewinnung von Promastigoten-Formen auf einen pH von 7 bis 7,5 mit einer Osmolarität von mindestens 300 Milliosmolen/kg Flüssigkeit und besonders 300 bis 380 Milliosmolen/kg Flüssigkeit gepuffert ist.

2. Verfahren nach Anspruch 1, **dadurch gekennzeichnet, daß** für die Kultur der Amastigoten-Stadien ein Medium 199H M verwendet wird.

3. Verfahren nach Anspruch 1 oder 2, **dadurch gekennzeichnet,, daß** zum Zeitpunkt der Verwendung dem Basismedium Antioxidantien, wie Hämin, Reduktionsmittel, wie reduziertes Glutathion, L-Cystein, sowie Vitamine oder Bathocuproinsulfonsaure zugesetzt werden.

4. Verfahren nach einem der Ansprüche 1 bis 3, **dadurch gekennzeichnet, daß** bezogen auf das Endmedium das Zellkulturmedium etwa 8 bis 15 % (V/V) bildet, die Aminosäuren im Verhältnis von 4 bis 8 % (G/V), die Zucker im Verhältnis von etwa 2 bis 4 % (G/V), die Antioxidantien, wie Hämin, im Verhältnis von 0,0002 bis 0,0015 % (G/V), das Glutathion im Verhältnis von 0,01 bis 0,05 % (G/V) und das L-Cystein im Verhältnis von 0,25 bis 0,50 % (G/V), die Bathocuproinsulfonsäure im Verhältnis von etwa 0,004 bis 0,008 % (V/V) vorliegen, wobei die Vitamine 1 % bis 5 % (V/V) bilden.

5. Verfahren nach Anspruch 4, **dadurch gekennzeichnet, daß** das Kulturmedium 10 % (V/V), die Aminosäuren 5 bis 6 % (G/V), die Zucker 2 bis 3 % (G/V), die Antioxidantien, wie Hämin, 0,0005 % (G/V), das Glutathion 0,025 % (G/V), das L-Cystein 3 % (G/V), die Bathocuproinsulfonsäure 0,005 % (V/V), die Vitamine 2 % (V/V) bilden.

6. Verfahren nach Anspruch 1, **dadurch gekennzeichnet, daß** für die Kultur von Promastigoten-Stadien ein Kulturmedium verwendet wird, das hergestellt ist ausgehend von einem Zellkulturmedium, wie dem Medium RPMI 1640, den Aminosäuren, wie L-Glutamin zugesetzt sind, wobei dieses Medium auf einen pH-Wert von 7 bis 7,5 gepuffert ist und ihm außerdem ein anderes, für die Kultur von Insektenzellen geeignetes Kulturmedium, speziell das Medium 199H M zugesetzt ist, das anorganische Salze, wie Hanks-Salze, und Antioxidantien, wie Hämin, enthält.

7. Verfahren nach Anspruch 6, **dadurch gekennzeichnet, daß** das Medium 199H M im Verhältnis von etwa mindestens 2 % (V/V) und das Rinderhämin von 0,0001 bis 0,0015 % (G/V) verwendet werden.

8. Verfahren nach Anspruch 7, **dadurch gekennzeichnet, daß** das Medium 199H M im Verhältnis von 2 % bis 10 % (V/V) und das Rinderhämin mit 0,0005 % (G/V) verwendet werden.

9. Verfahren nach einem der Ansprüche 1 bis 8, **dadurch gekennzeichnet, daß** Promastigoten-Formen der exponentialen Endphase in das Kulturmedium im Verhältnis von 10⁷ bis 10⁷ Promastigoten-Formen/ml des Mediums geimpft werden, die Adaptation und Kultur bei einer Temperatur in der Größenordnung von 28 bis 36°C und besonders um 32°C herum bei einem pH von 5,5 bis 6,5 durchgeführt werden, um eine vollständige Transformation der Promastigoten in Amastigoten zu erhalten.

10. Verfahren nach Anspruch 9 zur massiven und kontinuierlichen in vitro-Gewinnung von Promastigoten-Formen von Parasiten, **dadurch gekennzeichnet, daß** erhaltene Amastigoten-Formen im Verhältnis von 10⁶ bis 10⁷ Amastigoten/ml des Mediums geimpft werden und ihre Kultur bei einem pH von 7 bis 7,5 in einem klassischen Kulturmedium für die Entwicklung von Promastigoten-Formen aus Amastigoten erfolgt.

11. Verfahren nach Anspruch 10, **dadurch gekennzeichnet, daß** man Amastigoten-Formen am Ende des Exponentialphase verwendet.

12. Verfahren nach Anspruch 10 oder 11, **dadurch gekennzeichnet, daß** die erhaltenen Promastigoten-Formen für die Stufe des Impfens in ein Kulturmedium verwendet werden, um Amastigoten nach Anspruch 9 zu erzeugen.

13. Verfahren nach einem der vorangehenden Ansprüche, **dadurch gekennzeichnet, daß** die Amastigoten solche von T.cruzi sind und man Trypomastigoten-Formen an Stelle von Promastigoten-Formen benutzt.

14. Exkretions/Sekretions-Polypeptide, die direkt durch Konzentration und Dialyse ausgehend von Überständen von Kulturmedien von Amastigoten oder Promastigoten von Leishmanien oder T.cruzi erhalten wurden, die nach dem Verfahren eines der Ansprüche 1 bis 13gewonnen wurden.

15. Exkretions/Sekretions-Polypeptide nach Anspruch 14, **gekennzeichnet durch** Molekulargewichte (MG) in den Bereichen von 40 bis 60 kDa und 65 bis 90 kDa wie sie identifiziert werden **durch** Methionin-Markierung und Analyse der markierten Extrakte gemäß SDS-PAGE, jeweils für *L.infantum* und *L.amazonensis*.

16. Polyklonale Antikörper, die gegen Exkretions/Sekretions-Polypeptid-Antigene des Anspruchs 14 oder 15 gerichtet sind, die direkt ausgehend von den Überständen von Kulturmedien von Amastigoten oder Promastigoten von Leishmanien oder T.cruzi erhalten werden, die gemäß dem Verfahren eines der Ansprüche 1 bis 13 mit MG von 32 bis 50 kDa, 30 bis 36 kDa, 45 bis 60 kDa gewonnen wurden.

17. Monoklonale Antikörper, wie sie durch Fusion von myelomatösen Zellen mit Lymphozyten besonders der Milz oder Ganglien eines zuvor durch Injektion von Polypeptiden nach Anspruch 14 oder 15 immunisierten Tiers erhalten werden, Screening der Überstände der erhaltenen Hybridome, beispielsweise gemäß der E.L.I.S.A.- oder I.F.I.-Technik, um die spezifisch gegen eine parasitäre Form einer Spezies gerichteten Antikörper nachzuweisen, und Hybridomstämme, welche diese monoklonalen Antikörper sekretieren.

18. Monoklonale Antikörper, die bei der Maus gegen die Exkretions/Sekretions-Peptide von Amastigoten von *L.infantum, L.amazonensis* oder *T.cruzi* nach Anspruch 15 gebildet werden.

19. Verwendung als experimentelles Modell von parasitären Formen von Leishmanien und T.cruzi, die im infizierten Wirt vorhanden sind, besonders für das in vitro-Screening von Medikamenten und zum Testen der Empfindlichkeit gegen Medikamente, wobei das Screeningverfahren vorteilhafterweise umfaßt:
- das Inkontaktbringen von parasitaren Formen, wie sie nach dem Verfahren eines der Ansprüche 1 bis 13 kultiviert wurden, mit den zu testenden Produkten;
- den Einbau von Nukleotiden oder Aminosäuren, die mit einer radioaktiven Gruppe, beispielsweise tritiumhaltigem Thymidin markiert sind, zum Bestimmen der Aktivität der zu testenden Produkte oder zur Realisierung von Brauchbarkeitstests.

20. Kit zum Screenen von Produkten, die zur Behandlung von Parasitosen, besonders Leishmanien oder der Chagas-Krankheit verwendbar sein könnten, **dadurch gekennzeichnet, daß** er aufweist:
- einen Träger, wie eine Mehrlochplatte, welche parasitäre Formen umfaßt, wie sie nach dem Verfahren eines der Ansprüche 1 bis 13 erhalten werden,
- Reagenzien, um die Arzneimittelaktivität der getesteten Produkte an den parasitären Formen zu bestimmen.

21. Methode zum Nachweis in vitro einer Parasitose, besonders einer Leishmaniose oder der Chagas-Krankheit beim Menschen oder Tier oder ihr Nachweis und ihre Identifizierung beim Vektor-Insekt, durch Nachweis zirkulierender Antikörper, **dadurch gekennzeichnet, daß** die Methode umfaßt:
- Inkontaktbringen einer von dem zu untersuchenden Menschen oder Tier stammenden biologischen Probe mit einer parasitären Form, wie sie nach dem Verfahren eines der Ansprüche 1 bis 13 erhalten wurde, einem Exkretions/Sekretions-Polypeptid nach einem der Ansprüche 14 oder 15,
- Nachweis des gegebenenfalls gebildeten Immunkomplexes.

22. Kit zum Nachweis in vitro von Parasitosen nach Anspruch 21, **dadurch gekennzeichnet, daß** er aufweist:
- die Antigen-Reagenzien in immobilisierter Form, daß heißt die parasitären Formen, wie sie nach einem der Ansprüche 1 bis 13 erhalten werden, die Exkretions/Sekretions-Polypeptide nach Anspruch 14 oder 15,
- eine positive Kontrolle, die aus einem Serum mit bekanntem Titer besteht,
- eine negative Kontrolle, sowie
- Puffer und Reagenzien, die für die Entwicklung der Immunreaktion brauchbar sind.

23. Methode zum Nachweis in vitro einer Parasitose beim Menschen oder Tier besonders einer Infektion durch Leishmanien oder T.cruzi durch Nachweis von zirkulierenden Antigenen, **dadurch gekennzeichnet, daß** sie umfaßt:
- das Inkontaktbringen einer von dem zu untersuchenden Menschen oder Tier stammenden biologischen Probe mit Antikörpern nach einem der Ansprüche 16 oder 18 oder Fragmenten dieser Antikörper, die auf einem nicht immunogenen Träger immobilisiert sind, unter Bedingungen, die zur Bildung eines Antigen-Antikörperkomplexes geeignet sind,
- den Nachweis der Bildung des Komplexes.

24. Kit zum Nachweis in vitro einer Parasitose nach Anspruch 23, **dadurch gekennzeichnet, daß** er umfaßt:
- eine feste Phase, die als Träger für die Immunreaktion geeignet ist, wie eine Mikrotitrationsplatte,
- ein Antikörper-Präparat nach einem der Ansprüche 16 bis 18 oder von Fragmenten dieser Antikörper, die auf einem Träger immobilisiert sind, mit gegebenenfalls
- einer positiven Kontrolle, die aus einem Serum mit bekanntem Titer besteht,
- eine negative Kontrolle, sowie
- Puffer und Reagenzien, die für die Realisierung der Immunreaktion brauchbar sind und besonders der markierte homologe Antikörper.

25. Vakzin-Zusammensetzungen gegen Parasitosen, besonders Infektionen durch Leishmanien oder T.cruzi, **dadurch gekennzeichnet, daß** sie hergestellt sind ausgehend von Exkretions/Sektretions-Polypeptiden nach Anspruch 14 oder 15, Promastigoten-Formen, wie sie nach dem Verfahren eines der Ansprüche 1, 8 bis 10 erhalten werden, Amastigoten-Formen, wie sie nach dem Verfahren eines der Ansprüche 1 bis 9 und 11 oder 12 erhalten werden, in Kombination mit einem Vehikel und/oder einem Verabreichungshilfsstoff.

26. Vakzin-Zusammensetzungen nach Anspruch 25, **dadurch gekennzeichnet, daß** sie hergestellt sind ausgehend von Amastigoten von L.infantum oder L.chagasi.

27. Vakzin-Zusammensetzungen nach Anspruch 25 oder 26 für immunprophylaktische oder immuntherapeutische Zwecke.

## Claims

1. Method for the in vitro culture of different stages of tissular parasites chosen from the leishmanias and T.cruzi, **characterized in that** it is continuously produced, in a culture medium which is totally defined, single-phase, liquid, axenic and non-serous, free of macromolecules, i.e non-dialysable molecules at a cutoff point of 3 kDa, this culture medium comprising a medium for the culture of insect cells, to which is added inorganic salts of the Hanks salts type, amino acids such as L-glutamine, and sugars, buffered, in order to obtain amastigote forms, at a pH of 5.5 to 6.5 with an osmolarity of at least 400 milliosmoles/kg of liquid, and in particular 400 to 550 milliosmoles/kg of liquid, or at a pH of 7 to 7.5 for obtaining promastigote forms, with an osmolarity of at least 300 milliosmoles/kg of liquid, and in particular 300 to 380 milliosmoles/kg of liquid.

2. Method according to claim 1, **characterized in that** a 199H M medium is used for culturing the amastigote stages.

3. Method according to claim 1 or 2, **characterized in that** when used, antioxidants such as hemin, reducing agents, such as reduced glutathione, L-cysteine, as well as vitamins or bathocuproine disulphonic acid are added to the base medium.

4. Method according to any one of claims 1 to 3, **characterized in that**, relative to the final medium, the cell culture medium represents approximately 8 to 15% (V/V), the amino acids are present at a rate of 4 to 8% (W/V), the sugars at a rate of approximately 2 to 4% (W/V), the antioxidants, such as hemin, are present at a rate of 0.0002 to 0.0015% (W/V), the glutathione at a rate of 0.01 to 0.05%(W/V) and the L-cysteine at a rate of 0.25 to 0.50% (W/V), the bathocuproine disulphonic acid at a rate of approximately 0.004 to 0.008% (V/V), the vitamins representing 1% to 5%.

5. Method according to claim 4, **characterized in that** the culture medium represents 10% (V/V), the amino acids 5 to 6% (W/V), the sugars 2 to 3% (W/V), the anti-oxidizing agents, such as hemin, 0.0005% (W/V), the glutathione 0.025%, the L-cysteine 3% (W/V), the bathocuproine disulphonic acid 0.005% (V/V), the vitamins 2% (V/V).

6. Method according to claim 1, **characterized in that**, for the culture of promastigote stages, a culture medium is used produced from a cell culture medium, such as the RPMI 1640 medium, to which amino acids such as L-glutamine are added, this medium being buffered at a value of 7 to 7.5 and another culture medium also being added which is suitable for the culture of insect cells, in particular the 199H M medium, enclosing inorganic salts such as Hanks salts and antioxidants such as hemin.

7. Method according to claim 6, **characterized in that** the 199H M medium is used at a rate of approximately at least 2% (V/V), and bovine hemin from 0.0001% to 0.0015% (P/V).

8. Method according to claim 7, **characterized in that** the 199H M medium is used at a rate of 2% to 10% (V/V) and the bovine hemin of 0.0005% (P/V).

9. Method according to any one of claims 1 to 8, **characterized in that** the promastigote forms are cultured at the end of the exponential phase in said culture medium, at a rate of 10⁶ to 10⁷ promastigote forms/ml of medium, the adaptation and the culture are carried out at a temperature of the order of 28 to 36°C, and in particular around 32°C, at a pH of 5.5 to 6.5, in order to obtain a complete transformation of the promastigotes to amastiogotes.

10. Method according to claim 9, for the bulk and continuous in vitro production of promastigote forms, parasites **characterized by** the innoculation of the amastigote forms obtained at a rate of 10⁶ to 10⁷ amastigotes/ml of medium, their culture having a pH of 7 to 7.5 in a standard culture medium for the development of the promastigote forms to amastigotes.

11. Method according to claim 10, **characterized in that** the amastigote forms are used at the end of the exponential phase.

12. Method according to claim 10 or 11, **characterized in that** the promastigote forms obtained are used for the innoculation stage in a culture medium for producing the amastigotes according to claim 9.

13. Method according to one of the previous claims, **characterized in that** the amastigotes are from T.cruzi and trypomastigote forms are used instead of promastigote forms.

14. The excretion/secretion polypeptides obtained directly by concentration and dialysis from the supernatant of the leishmanias or T.cruzi amastigote or promastigote culture media produced according to the method according to any one of claims 1 to 13.

15. Excretion/secretion polypeptides according to claim 14, **characterized by** MWs in the regions 40 to 60 kDa and 65 to 90 kDa such as those identified by methionine labelling and analysis of the SDS-PAGE labelled extracts respectively for *L.infantum* and *L.amazonensis*.

16. Polyclonal antibodies directed against excretion/secretion antigenic polypeptides according to claim 14 or 15, obtained directly from the supernatants of the leishmanias or T.cruzi amastigote or promastigote culture media produced according to the method of one of claims 1 to 13 having MWs of 32 to 50 kDa, 30 to 36 kDa, 45 to 60 kDa.

17. Monoclonal antibodies as obtained by fusion of myelomatous cells with the lymphocytes in particular of the spleen or the ganglions of an animal previously immunized by injection of the polypeptides according to claim 14 or 15, screening of the supernatants of the hybridoma obtained, for example according to the E.L.I.S.A. or I.F.I. technique so as to reveal the antibodies directed specifically against a parasitic form of a species, and hybridoma strains secreting these monoclonal antibodies.

18. Monoclonal antibodies generated in the mouse against the excretion/secretion peptides of amastigotes of *L.infantum, L.amazonensis* or *T.cruzi* according to claim 15.

19. Use as an experimental model of the leishmanias or T.cruzi parasitic forms present in the infected host, in particular for the in vitro screening of medicaments and for testing the sensitivity to medicaments, the screening method advantageously comprising:
- bringing into contact with the products to be tested of the parasitic forms as cultured according to the method of one of claims 1 to 13.
- incorporation of the nucleotides or amino acids labelled with a radioactive group, for example tritiated thymidine for determining the activity of the products to be tested or the realization of viability tests.

20. Kit for the screening of products capable of being used for the treatment of parasitosis, more particularly leishmaniasis or Chagas' disease, **characterized in that** it comprises:
- a support such as a multi-well plate containing the parasitic forms as obtained by the method according to one of claims 1 to 13,
- the reagents for determining the medicinal activity of products tested on the parasitic forms.

21. Method for the in vitro detection of a parasitosis, in particular a leishmaniasis or Chagas disease, in a human or animal, or their detection and identification in the vector insect, by detection of the circulating antibodies, **characterized in that** it comprises:
- the bringing in contact of a biological sample originating from the human or animal to be examined with a parasitic form as obtained by the method according to any one of claims 1 to 13, an excretion/secretion polypeptide according to claim 14 or 15,
- the detection of the immunological complex possibly formed.

22. Kit for the in vitro detection of parasitosis according to claim 21, **characterized in that** it comprises:
- the antigenic reagents in immobilized form, namely the parasitic forms as obtained according to one of claims 1 to 13, the excretion/secretion polypeptides according to claim 14 or 15,
- a positive control, constituted by a serum of known titre,
- a negative control as well as
- the buffers and reagents which can be used to reveal the immunological reaction.

23. Method for the in vitro detection of a parasitosis in a human or animal, in particular an infection by leishmanias or T.cruzi, by detecting the circulating antigens, **characterized in that** it comprises:
- the bringing into contact of a biological sample originating from the human or animal to be examined with the antibodies according to one of claims 16 to 18, or fragments of these antibodies, immobilized on a non-immunogenic support under the conditions suitable for the production of an antigen-antibody complex,
- the detection of the formation of the complex.

24. Kit for the in vitro detection of a parasitosis according to claim 23, **characterized in that** it comprises:
- a suitable solid phase serving as support for the immunological reaction, such as a microtitration plate,
- an antibody preparation according to one of claims 16 to 18, or fragments of these antibodies immobilized on a support with, if appropriate,
- a positive control constituted by a serum of known titre,
- a negative control, as well as
- buffers and reagents which can be used to carry out the immunological reaction, and in particular the labelled homologous antibody.

25. Compositions of vaccines against parasitosis, in particular infections by leishmanias or T. cruzi, **characterized in that** they are produced from excretion/secretion polypeptides according to claim 14 or 15, promastogote forms as obtained according to the method of any one of claims 1, 8 to 10, amastigote forms as obtained according to the method of any one of claims 1 to 9 and 11 or 12, in combination with a vehicle and/or an administration adjuvant.

26. Compositions of vaccines according to claim 25, **characterized in that** they are produced from amastigotes of L. infantum or L. chagasi.

27. Compositions of vaccines according to claim 25 or 26, for immunoprophylactic or immunotherapeutic purposes.
